# EUROPÄISCHE PATENTSCHRIFT

(11) **EP 2 706 124 B1**
(45) Veröffentlichungstag und Bekanntmachung des Hinweises auf die Patenterteilung: **22.03.2017**
(21) Anmeldenummer: 13004290.6
(22) Anmeldetag: 30.08.2013
(51) Int. Cl.: C12Q 1/68

(54) **Zeitgleicher Nachweis verschiedener Micro-RNA-Biogenese-Formen**
Simultaneous detection of different micro RNA biogenesis forms
Mise en évidence simultanée de différentes micro-formes de biogenèse RNA

(30) Priorität: 07.09.2012 DE 102012215925
(43) Veröffentlichungstag der Anmeldung: 12.03.2014
(73) Patentinhaber: Fraunhofer-Gesellschaft zur Förderung der angewandten Forschung e.V., 80686 München (DE)
(72) Erfinder: Weishaupt, Sonja, 88263 Horgenzell (DE); Lemuth, Karin, 70195 Stuttgart (DE); Rupp, Steffen, 70569 Stuttgart (DE)
(74) Vertreter: Schrell, Andreas

(56) Entgegenhaltungen:
- EP-A1- 2 336 354
- WO-A2-2010/120853
- US-A1- 2005 260 648
- US-A1- 2012 021 420
- NICOLE C HAUSER ET AL: "Utilising the left-helical conformation of L-DNA for analysing different marker types on a single universal microarray platform", NUCLEIC ACIDS RESEARCH, OXFORD UNIVERSITY PRESS, GB, Bd. 34, Nr. 18, 1. Oktober 2006 (2006-10-01), Seiten 5101-5111, XP002667723, ISSN: 1362-4962, DOI: 10.1093/NAR/GKL671
- VORWERK S ET AL: "Microfluidic-based enzymatic on-chip labeling of miRNAs", NEW BIOTECHNOLOGY, ELSEVIER BV, NL, Bd. 25, Nr. 2-3, 1. Oktober 2008 (2008-10-01), Seiten 142-149, XP025711668, ISSN: 1871-6784, DOI: 10.1016/J.NBT.2008.08.005 [gefunden am 2008-08-20]
- SCHMITTGEN ET AL: "Real-time PCR quantification of precursor and mature microRNA", METHODS : A COMPANION TO METHODS IN ENZYMOLOGY, ACADEMIC PRESS INC., NEW YORK, NY, US, Bd. 44, Nr. 1, 23. Dezember 2007 (2007-12-23), Seiten 31-38, XP022398577, ISSN: 1046-2023, DOI: 10.1016/J.YMETH.2007.09.006
- Lorenzo F Sempere: "Recent advances in miRNA-based diagnostic applications", EXPERT REVIEW OF MOLECULAR DIAGNOSTICS, vol. 12, no. 6, 1 July 2012 (2012-07-01), pages 557-559, XP055296614, GB ISSN: 1473-7159, DOI: 10.1586/erm.12.47

## Beschreibung

Die vorliegende Erfindung betrifft ein Verfahren zum differenzierten Nachweis von in einer Probenlösung vorhandenen, mindestens zwei unterschiedlichen ersten und zweiten RNA-Molekülen, die jeweils eine identische Polynucleotidsequenz A aufweisen, und einen Kit zur Durchführung dieses Verfahrens.

In den letzten paar Jahren wurde die Bedeutung von mikroRNAs, einer Klasse nichtkodierender kleiner RNAs, bei der Tumorentstehung erkannt. mikroRNAs spielen allgemein eine wichtige Rolle bei der Regulation der Genexpression durch Abbau von einer mRNA beziehungsweise Unterdrückung der Translation. Sie sind auch an einer Vielzahl essentieller biologischer Prozesse in Eukaryoten beteiligt, unter anderem am Zellwachstum, der Differenzierung und der Apoptose von Zellen sowie an der Entstehung von Tumoren.

Bei der mikroRNA-Biogenese wird eine mehrere tausend Nucleotide umfassendes Primärtranskript (pri-mikroRNA) mit Haarnadel-Struktur (auch als "hair pin" oder "stem-loop" bezeichnet) im Zellkern zunächst zu einer 60 bis 110 Nucleotide umfassenden Vorläufer ("precursor")- mikroRNA (pre-mikroRNA) ebenfalls mit Haarnadel-Struktur und diese im Cytosol weiter zu einer 20 bis 22 Nucleotid langen aktiven reifen ("mature") mikroRNA prozessiert.

MikroRNAs stellen eine neue und vielversprechende Klasse von Tumormarkern dar, die ein hohes Potential zur Sub-Klassifizierung von insbesondere komplexen Tumoren haben. In Tumoren wurde ein verändertes Expressionslevel der verschiedenen mikroRNA-Formen, nämlich der Vorläufer- und reifen Formen, beobachtet. Dies ist durch die Fehlregulation ihrer Expression verursacht. So zeigen nicht nur die regulatorisch aktiven reifen mikroRNAs, sondern auch die Vorläufer-mikroRNAs tumorbasierte Expressionsunterschiede. Beispielsweise wurde bei Lungenkrebs ein abnormales Verhältnis in der Expression von Vorläufer- und reifen mikroRNAs beschrieben.

Daher ist es von großem Interesse, die verschiedenen mikroRNA-Formen detektieren zu können. Eine entsprechende Detektion gestaltet sich als schwierig, da die Vorläufer-mikroRNA als Haarnadel-Struktur vorliegt, aus der die reife Form prozessiert wird und so die komplette Nucleotidsequenz der 20 bis 22 Nucleotide umfassende reifen mikroRNA identisch in der 60 bis 110 Nucleotide umfassenden Vorläufer-mikroRNA vorliegt. Die alleinige Verwendung einer zur reifen mikroRNA komplementären Sonde zur differenzierten Detektion von reifen und Vorläufer-mikroRNA scheidet somit aus. Die übrigen, nicht zur Haarnadel-Struktur gehörenden Basensequenzen erlauben meist keine Detektion mittels molekularer Methoden, da sie für die Diskriminierung nicht ausreichend lang sind. Dies erweist sich als Nachteil für ein Verfahren auf Hochdurchsatzbasis zum differenzierten, insbesondere zeitgleichen, Nachweis von einer in einer Probenlösung vorhandenen Vorläufer- und reifen mikroRNAs aus einer RNA-Probe mittels eines DNA-Mikroarrays.

Bisher wird zur Detektion von reifen mikroRNA und/oder Vorläufer-mikroRNA aus Zellen bevorzugt die klassische Northern Blot-Methode verwendet. Der wesentliche Nachteil dieser Methode liegt darin, dass sie sehr zeitaufwendig ist und somit keine Hochdurchsatzanalysen möglich sind (Cissell et al., Trends in mikroRNA detection, Anal Bional Chem, 2009, 394(4), 1109-1116).

Eine weitere, weit verbreitete mikroRNA-Detektionsmethode stellt die quantitative Real-Time PCR (qRT-PCR) dar. Auch bei dieser Methode ist kein Hochdurchsatz möglich. Der differenzierten Nachweis kann nur bei wenigen mikroRNAs zeitgleich erfolgen (De Planell-Saguer et. al, Anal Chim Acta, 2011, 699(2), 134-152; Farazi et al., J. Pathol, 2011, 223(2), 102-115).

Weiterhin ist die Vergleichbarkeit verschiedener Tests unter anderem von der PCR-Effizienz abhängig. Dies erschwert den Vergleich verschiedener mikroRNAs in einer Probe (Bustin et al., Clin Chem, 2009, 55(4), 611-622; Balcells et al., BMC Biotechnol., 2011, 11, 70). Außerdem ist die Auswahl an kommerziellen Systemen limitiert, bei denen reifen und Vorläufer-mikroRNA auf qRT-PCR-Basis nachgewiesen werden können (beispielsweise miScript PCR System; Qiagen, Hilden, Deutschland).

Die Detektion von reifen und Vorläufer-mikroRNA im Hochdurchsatz, das heißt insbesondere auf der Basis einer DNA-Mikroarray-Technologie, erfolgt bislang auf einer der Analyse vorangegangenen Trennung von reifen und Vorläufer-mikroRNA aufgrund ihrer unterschiedlichen Größe. Hierzu werden die ungefähr 20 Nucleotide umfassenden reifen mikroRNAs und die 60 bis 110 Nucleotide umfassenden Vorläufer-mikroRNAs mittels klassischer denaturierender Polyacrylamid-Gel-Elektrophorese aufgetrennt, die entsprechenden Fraktionen ausgeschnitten, eluiert und damit separiert. Ein derartiges Verfahren ist sehr zeitintensiv. Zudem kann eine 100 %ige Trennung von reifen und Vorläufer-mikroRNA nicht gewährleistet werden.

Eine zeitsparende Alternative hierzu ist das "flashPAGE"-Fractionator System von Ambion, Austin, USA. Dieses System ist sehr kostenintensiv und basiert ebenfalls auf der Auftrennung der reifen und Vorläufer-mikroRNA enthaltenen Fraktionen (Li et al., Anal Bioanal Chem, 2009, 394(4), 1117-1124). Nach der Trennung dieser beiden Fraktionen findet eine Analyse mittels DNA-Mikroarrays in separaten Hybridisierungen statt, was den unmittelbaren Vergleich der beiden mikroRNA-Formen untereinander erschwert.

Ebenso werden im Stand der Technik DNA-Mikroarrays verwendet, welche sowohl spezifische Sonden für reife mikroRNA als auch für den Haarnadel-Bereich der Vorläufer-mikroRNA aufweisen. Die spezifischen Sonden für die reifen mikroRNAs können an beide Formen der mikroRNA, das heißt an die reifen und die Vorläufer-mikroRNA binden. Die spezifischen Sonden für den Haarnadel-Bereich der Vorläufer-mikroRNA können allein die Vorläufer-mikroRNA detektieren (GeneChip miRNA 2.0 Array; Affymetrix, Santa Clara, USA; GenoExplorer mikroRNA sytem; GenoSensor Corporation, Tempe, USA). Hierbei muss jedoch eine Datenanalyse durch Verrechnung der Signale der beiden Sonden auf Basis einer mathematischen Näherung erfolgen. Die Platzierung einer spezifischen Sonde im Haarnadel-Bereich der Vorläufer-mikroRNA - in Abhängigkeit der Lage der reifen mikro-RNA Sequenz in der Vorläufer-mikroRNA - ist aufgrund der notwendigen Anzahl an Nucleotiden zur spezifischen Anbindung und ausreichenden Diskriminierung limitiert. Von 60 bis 110 Nucleotiden der Vorläufer-mikroRNA sind nämlich etwa 40 bis 44 Nucleotide identisch zur reifen mikroRNA.

Das der Erfindung zugrunde liegende technische Problem liegt daher unter anderem darin, Verfahren und Mittel bereitzustellen, die insbesondere die vorgenannten Nachteile zu überwinden, insbesondere ein Verfahren zum differenzierten Nachweis von in einer Probenlösung vorhandenen, mindestens zwei unterschiedlichen ersten und zweiten RNA-Molekülen bereitzustellen, insbesondere durch das Verfahren das molare Verhältnis zwischen diesen zwei unterschiedlichen ersten und zweiten RNA-Molekülen zu bestimmen, und einen Kit zur Durchführung dieser Verfahren bereitzustellen. In besonders wünschenswerter Weise sollen die bereitzustellenden Verfahren und Mittel einen zeitgleichen Nachweis der unterschiedlichen RNA-Moleküle in differenzierender Weise ermöglichen. In besonders wünschenswerter Weise soll ein solches Verfahren ein Hochdurchsatzverfahren sein, das heißt in effizienter und kostengünstiger Weise eine schnelle Analyse einer großen Zahl an Proben ermöglichen.

Dieses technische Problem wird insbesondere durch die Lehre der unabhängigen Ansprüche gelöst.

Erfindungsgemäß ist das technische Problem insbesondere durch ein Verfahren zum differenzierten Nachweis von in einer Probenlösung vorhandenen, mindestens zwei unterschiedlichen ersten und zweiten RNA-Molekülen gelöst, die jeweils eine identische Polynucleotidsequenz A aufweisen, wobei das Verfahren folgende Schritte umfasst:
a) Bereitstellen einer Probenlösung, die die mindestens zwei unterschiedlichen ersten und zweiten RNA-Moleküle aufweist, wobei das erste RNA-Molekül die Polynucleotidsequenz A ist und das zweite RNA-Molekül die Polynucleotidsequenz A und unmittelbar anschließend 5'-wärts ein Nucleotidelement P aufweist,
b) Bereitstellen eines eine Primernukleotidsequenz cA und eine cZIP X-Nukleotidsequenz aufweisenden Primer-cZIP X-Polynucleotids, wobei die Primernukleotidsequenz cA komplementär zu einem endständigen 5'-Abschnitt der Polynucleotidsequenz A ist, und eines die Primernukleotidsequenz cA und eine cZIP Y-Nukleotidsequenz aufweisenden Primer-cZIP Y-Polynucleotids,
c) Bereitstellen eines Nucleotid-Mikroarrays umfassend mindestens zwei unterschiedliche erste und zweite auf einem Nucleotid-Mikroarrayträger an unterschiedlichen Positionen immobilisierte Gegen-ZIPs, wobei das erste Gegen-ZIP komplementär zu der cZIP X-Nucleotidsequenz und das zweite Gegen-ZIP komplementär zu der cZIP Y-Nucleotidsequenz ist,
d) Adenylierung der in der Lösung aus Schritt a) vorhandenen ersten und zweiten RNA-Moleküle, wobei eine erste und zweite adenylierte RNA-Moleküle aufweisende Lösung erhalten wird,
e) Teilen der die ersten und zweiten adenylierten RNA-Moleküle aufweisenden Lösung aus Schritt d) in eine erste Lösung und eine zweite Lösung,
f) Versetzen der ersten Lösung mit dem in Schritt b) bereitgestellten Primer-cZIP X-Polynucleotid und Durchführung einer Hybridisierung mit dem ersten und zweiten adenylierten RNA-Molekül, wobei eine Lösung A1 erhalten wird,
g) Versetzen der zweiten Lösung mit dem in Schritt b) bereitgestellten Primer cZIP Y-Polynucleotid und Durchführung einer Hybridisierung mit dem ersten und zweiten adenylierten RNA-Molekül, wobei eine Lösung B1 erhalten wird,
h) Versetzen der in Schritt f) erhaltenen Lösung A1 mit mindestens einem ersten Nucleosidtriphosphat, das mit einem Fluoreszenzfarbstoff markiert ist, wobei das erste Nucleosidtriphosphat ausgewählt ist aus desoxy-Thymidintriphosphat und desoxy-Uridintriphosphat, und Durchführung einer enzymatischen Strangverlängerung des mit den ersten und zweiten adenylierten RNA-Molekülen hybridisierten Primer-cZIP X-Polynukleotids, wobei eine Lösung A2 erhalten wird,
i) Versetzen der in Schritt g) erhaltenen Lösung B1 mit mindestens einem zweiten Nucleosidtriphosphat, das mit einem Fluoreszenzfarbstoff markiert ist, und wobei das zweite Nucleosidtriphosphat ausgewählt ist aus der Gruppe bestehend aus desoxy-Guanosintriphosphat, desoxy-Adenosintriphosphat und desoxy-Cytidintriphosphat, und Durchführung einer enzymatischen Strangverlängerung des mit den ersten und zweiten adenylierten RNA-Molekülen hybridisierten Primer-cZIP Y-Polynucleotids, wobei eine Lösung B2 erhalten wird,
j) Inkontaktbringen der in den Schritten h) und i) erhaltenen Lösungen A2 und B2 mit dem in Schritt c) bereitgestellten Nucleotid-Mikroarray und Durchführung einer Hybridisierung der Primer-cZIP X- und Primer-cZIP Y-Polynucleotide mit den ersten und zweiten Gegen-ZIPs und
k) differenziertes Nachweisen der mindestens zwei unterschiedlichen ersten und zweiten RNA-Moleküle durch eine Fluoreszenzdetektion.

Die vorliegende Erfindung betrifft ein erfindungsgemäßes Verfahren, wobei das zweite RNA-Molekül die Polynucleotidsequenz A 3'-wärts zu dem Nucleotidelement P aufweist, und wobei die Primernukleotidsequenz cA komplementär zu dem 5'-Abschnitt der Polynucleotidsequenz A ist.

Die vorliegende Erfindung betrifft in bevorzugter Ausführungsform ein erfindungsgemäßes Verfahren, wobei die in den Schritten h) und i) erhaltenen Lösungen A2 und B2 vor Schritt j) vereinigt werden, so dass eine vereinigte Lösung C erhalten wird, die in Schritt j) mit dem Nucleotid-Mikroarray in Kontakt gebracht wird.

Die vorliegende Erfindung betrifft in bevorzugter Ausführungsform ein erfindungsgemäßes Verfahren, wobei die mindestens zwei unterschiedlichen ersten und zweiten RNA-Moleküle mikroRNA-Moleküle sind, insbesondere Sinn-Strang (sense-strand) mikroRNA-Moleküle.

Die vorliegende Erfindung betrifft in bevorzugter Ausführungsform ein erfindungsgemäßes Verfahren, wobei das erste RNA-Molekül eine reife RNA, insbesondere eine reife mikroRNA, und das zweite RNA-Molekül eine Vorläufer-RNA, insbesondere Vorläufer-mikroRNA, ist, z. B. eine pri- oder pre-mikroRNA.

Die vorliegende Erfindung betrifft in bevorzugter Ausführungsform ein erfindungsgemäßes Verfahren, wobei das Primer-cZIP X-Polynucleotid und das Primer-cZIP Y-Polynucleotid eine DNA, insbesondere eine D-DNA, ist.

Die vorliegende Erfindung betrifft in bevorzugter Ausführungsform ein erfindungsgemäßes Verfahren, wobei zur Bereitstellung der Probenlösung gemäß Schritt a) eine Amplifikation des ersten und zweiten RNA-Moleküls erfolgt.

Die vorliegende Erfindung betrifft in bevorzugter Ausführungsform ein erfindungsgemäßes Verfahren, wobei während oder nach der Adenylierung gemäß Schritt d) eine Amplifikation der mindestens zwei unterschiedlichen ersten und zweiten adenylierten RNA-Moleküle erfolgt.

Die vorliegende Erfindung betrifft in bevorzugter Ausführungsform ein erfindungsgemäßes Verfahren, wobei im Anschluss an die Durchführung der Hybridisierung gemäß der Schritte f) und g) und die Strangverlängerung gemäß der Schritte h) und i) eine Denaturierung der erhaltenen strangverlängerten Nucleinsäurehybride, besonders bevorzugt ein Abbau des ersten und zweiten adenylierten RNA-Moleküls, bevorzugt mittels einer Ribonuklease, insbesondere der Ribonuklease H, durchgeführt wird.

Die vorliegende Erfindung betrifft in bevorzugter Ausführungsform ein erfindungsgemäßes Verfahren, wobei das erste und das zweite Nucleosidtriphosphat mit demselben Fluoreszenzfarbstoff markiert sind.

Die vorliegende Erfindung betrifft in bevorzugter Ausführungsform ein erfindungsgemäßes Verfahren, wobei der Fluoreszenzfarbstoff ein Cyaninfarbstoff, insbesondere Cy3, ist.

Die vorliegende Erfindung betrifft auch einen Kit zur Durchführung eines erfindungsgemäßen Verfahrens zum differenzierten Nachweis von in einer Probenlösung vorhandenen, mindestens zwei unterschiedlichen ersten und zweiten RNA-Molekülen, die jeweils eine identische Polynucleotidsequenz A aufweisen, enthaltend:
a1) einen Nucleotid-Mikroarray umfassend mindestens zwei unterschiedliche erste und zweite auf dem Nucleotid-Mikroarrayträger an unterschiedlichen Positionen immobilisierte Gegen-ZIPs,
b1) ein eine Primernukleotidsequenz cA und eine cZIP X-Nukleotidsequenz aufweisendes Primer-cZIP X-Polynucleotid, wobei die Primernukleotidsequenz cA komplementär zu einem endständigen 5'-Abschnitt der Polynucleotidsequenz A ist und wobei die cZIP X-Nukleotidsequenz komplementär zu dem ersten Gegen-ZIP des Nucleotid-Mikroarrays ist, und
c1) ein die Primernukleotidsequenz cA und eine cZIP Y-Nukleotidsequenz aufweisendes Primer-cZIP Y-Polynucleotid, wobei die cZIP Y-Nukleotidsequenz komplementär zu dem zweiten Gegen-ZIP des Nucleotid-Mikroarrays ist und
wobei der Kit eine Lösung I enthält, die mindestens ein mit einem Fluoreszenzfarbstoff markiertes erstes Nucleosidtriphosphat und eine Lösung II, die mindestens ein mit einem Fluoreszenzfarbstoff markiertes zweites Nucleosidtriphosphat enthält, wobei das erste Nucleosidtriphosphat ausgewählt ist aus der Gruppe bestehend aus desoxy-Uridintriphosphat und desoxy-Thymidintriphosphat und wobei das zweite Nucleosidtriphosphat ausgewählt ist aus der Gruppe bestehend aus desoxy-Guanosintriphosphat, desoxy-Adenosintriphosphat und desoxy-Cytidintriphosphat.

Die vorliegende Erfindung betrifft in bevorzugter Ausführungsform einen erfindungsgemäßen Kit, wobei der Kit mindestens ein Enzym für eine Polyadenylierung und mindestens ein Enzym für die Strangverlängerung enthält.

In bevorzugter Ausführungsform der vorliegenden Erfindung weist der erfindungsgemäße Kit in der Lösung I und Lösung II zusätzlich mindestens ein unmarkiertes Nucleosidtriphosphat ausgewählt aus der Gruppe bestehend aus desoxy-Guanosintriphosphat, desoxy-Adenosintriphosphat, desoxy-Cytidintriphosphat, desoxy-Uridintriphosphat und desoxy-Thymidintriphosphat auf, insbesondere weist der Kit mindestens ein unmarkiertes Nucleosidtriphosphat ausgewählt aus der Gruppe bestehend aus desoxy-Uridintriphosphat und desoxy-Thymidintriphosphat auf.

In einer bevorzugten Ausführungsform der vorliegenden Erfindung weist der erfindungsgemäße Kit in der Lösung I zusätzlich mindestens ein unmarkiertes Nucleosidtriphosphat ausgewählt aus der Gruppe bestehend aus desoxy-Uridintriphosphat und desoxy-Thymidintriphosphat auf.

In einer bevorzugten Ausführungsform der vorliegenden Erfindung weist der erfindungsgemäße Kit in der Lösung I als Nucleosidtriphosphate ausschließlich mit einem Fluoreszenzfarbstoff markierte und/oder unmarkierte Nucleosidtriphosphate auf, ausgewählt aus der Gruppe bestehend aus desoxy-Uridintriphosphat und desoxy-Thymidintriphosphat.

In einer bevorzugten Ausführungsform der vorliegenden Erfindung weist der erfindungsgemäße Kit in der Lösung II zusätzlich mindestens ein, bevorzugt mindestens 4 unmarkierte Nucleosidtriphosphate ausgewählt aus der Gruppe bestehend aus desoxy-Guanosintriphosphat, desoxy-Adenosintriphosphat, desoxy-Cytidintriphosphat, desoxy-Uridintriphosphat und desoxy-Thymidintriphosphat auf.

In einer bevorzugten Ausführungsform der vorliegenden Erfindung weist der erfindungsgemäße Kit in der Lösung II mindestens unmarkiertes desoxy-Guanosintriphosphat, unmarkiertes desoxy-Adenosintriphosphat, unmarkiertes desoxy-Cytidintriphosphat und mindestens ein weiteres unmarkiertes Nucleosidtriphosphat auf, ausgewählt aus der Gruppe bestehend aus desoxy-Uridintriphosphat und desoxy-Thymidintriphosphat.

Mit dem erfindungsgemäßen Verfahren kann eine Aussage darüber getroffen werden, ob das erste RNA-Molekül und/oder das zweite RNA-Molekül in der Probenlösung vorhanden ist. Insbesondere kann damit das quantitative Verhältnis zwischen den ersten und den zweiten RNA-Molekülen in der Probenlösung bestimmt werden. Das erfindungsgemäße Verfahren erbringt den erwünschten differenzierten Nachweis, ohne dass die beiden ersten und zweiten RNA-Molekülen aufgrund ihrer Größe oder Polarität voneinander getrennt werden müssen. Das erfindungsgemäße Verfahren ist für den Hochdurchsatz geeignet. Mittels des erfindungsgemäßen Verfahrens ist insbesondere die Differenzierung aller in einem Organismus vorkommenden mikroRNAs möglich.

Ein wesentlicher Vorteil der erfindungsgemäßen Verfahrensweise liegt darin, dass diese einen zeitgleichen Nachweis von reifer und Vorläufer-mikroRNA aus vorzugsweise ein und derselben RNA-Probe, insbesondere in einem Hochdurchsatzsystem, zum Beispiel einem DNA-Mikroarray-basierten System, ermöglicht. Durch die Verwendung eines identischen mikroRNA-spezifischen Bereichs, nämlich der Primer-Nucleotidsequenz cA für beide mikroRNA-Typen, werden sondenspezifische Unterschiede vermieden. Eine mögliche Verzerrung durch unterschiedlichen Farbstoffeinbau kann darüber hinaus durch die bevorzugte alleinige Verwendung von dem Cyaninfarbstoff, insbesondere Cy3, als Fluoreszenzfarbstoff in sogenannten "one colour"-Experimenten unterbunden werden. Im Rahmen einer bevorzugt vorgesehenen Amplifikation ist zudem die Analyse mengenlimitierter Proben, zum Beispiel Biopsien, möglich. Die vorliegende Erfindung stellt sich daher besonders vorteilhaft im Bereich der medizinischen und klinischen Diagnostik dar und ermöglicht eine verbesserte Tumorforschung, beispielsweise eine verbesserte Klassifizierung und Identifizierung von heterogenen, aggressiven und bösartigen Tumoren, beispielsweise Lymphomen, als Basis für eine erfolgsversprechende Therapie im Rahmen einer beispielsweise individualisierten Medizin.

Das erfindungsgemäße Verfahren weist einen Schritt a) auf, wobei eine Probenlösung bereitgestellt wird, die die mindestens zwei unterschiedlichen RNA-Moleküle, aufweist. Die Probenlösung ist bevorzugt eine wässrige Lösung.

Die Probe selbst kann aus einem beliebigen biologischen System stammen, beispielsweise Biopsie-Material, Zellen, Zellverbänden, Gewebe, Viren, Bakterien, Pilze, Parasiten, Bodenproben, Sperma, Speichel oder Hautproben oder dergleichen.

Das erste RNA-Molekül umfasst eine Polynucleotidsequenz A. Das erste RNA-Molekül besteht insbesondere aus der Polynucleotidsequenz A.

In einer bevorzugten Ausführungsform weist die Polynucleotidsequenz A mindestens fünf Nucleotide, bevorzugt mindestens acht Nucleotide, bevorzugt mindestens zwölf Nucleotide, bevorzugt mindestens fünfzehn Nucleotide, bevorzugt mindestens achtzehn Nucleotide, bevorzugt mindestens zwanzig Nucleotide, bevorzugt 5 bis 50 Nucleotide, bevorzugt 10 bis 40 Nucleotide, bevorzugt 15 bis 30 Nucleotide, bevorzugt 18 bis 25 Nucleotide, bevorzugt 20 bis 22 Nucleotide auf.

Erfindungsgemäß weisen die in der gemäß Schritt a) bereitgestellten Probenlösung vorhandenen mindestens zwei unterschiedlichen ersten und zweiten RNA-Moleküle eine identische Polynucleotidsequenz A und unterscheiden sich dadurch, dass das zweite RNA-Molekül zusätzliche Nucleotide, insbesondere mindestens einen zusätzlichen Polynucleotidabschnitt, aufweist.

Das zweite RNA-Molekül weist erfindungsgemäß die Polynucleotidsequenz A und unmittelbar anschließend ein Polynucleotidelement P auf, wobei sich das Polynucleotidelement P 5'-wärts zu der Polynucleotidsequenz A befindet.

In einer bevorzugten Ausführungsform weist das Nucleotidelement P mindestens ein Nucleotid, bevorzugt mindestens zwei Nucleotide, bevorzugt mindestens fünf Nucleotide, bevorzugt mindestens zehn Nucleotide, bevorzugt mindestens fünfzehn Nucleotide, bevorzugt mindestens zwanzig Nucleotide, bevorzugt mindestens fünfundzwanzig Nucleotide auf.

In bevorzugter Ausführungsform liegt das erste, das zweite oder beide RNA-Moleküle als Sinn(sense)-Strang, das heißt als sense-strand oder sense-RNA-Molekül, vor.

In einer bevorzugten Ausführungsform sind die mindestens zwei unterschiedlichen ersten und zweiten RNA-Moleküle mikroRNA-Moleküle.

In einer bevorzugten Ausführungsform ist das erste RNA-Molekül eine reife mikroRNA und das zweite RNA-Molekül eine Vorläufer-mikroRNA, wobei die Vorläufer-mikroRNA eine ursprünglich in der Zelle vorkommende mikroRNA und/oder eine im Cytosol vorkommende Vorläufer-mikroRNA ist. Im Zusammenhang mit der vorliegenden Erfindung wird unter einer Vorläufer-RNA eine nicht-reife RNA verstanden, insbesondere eine im Verlauf der Transkription und Prozessierung gebildete Zwischen-RNA-Form, zum Beispiel ein primäres Transkript (pri-RNA, insbesondere pri-miRNA) oder eine Vorläufer-RNA im engeren Sinne (pre-RNA, insbesondere pre-miRNA).

In einer bevorzugten Ausführungsform der vorliegenden Erfindung werden vor oder während Schritt a) die ersten und zweiten RNA-Moleküle, insbesondere mikroRNAs, aus biologischem Material, z. B. Zellen, Zellteilen, Pilzen, Gewebe, Biopsie-Material, Viren, Probenmaterialien, oder dergleichen, isoliert und liegen vorzugsweise in isolierter Form vor, so dass sie anschließend in Lösung bereitgestellt werden können. Anschließend können in bevorzugter Ausführungsform die erhaltenen RNA-Moleküle angereichert, z. B. mittels säulenbasierter Methoden, insbesondere mit dem mirPremier mikroRNA Isolation Kit, Sigma-Aldrich, Taufkirchen, Deutschland, und in Lösung, z. B. gepufferter Lösung, bereitgestellt werden.

In bevorzugter Ausführungsform werden die, vorzugsweise aus biologischem Material bereitgestellten oder gewonnenen, ersten und zweiten RNAs, vorzugsweise mikroRNAs, enzymatisch amplifiziert.

In einem Schritt b) werden Primer zur Identifizierung der Polynucleotidsequenz A in dem ersten und zweiten RNA-Molekül bereitgestellt. Diese Primer weisen einerseits eine zu der Polynucleotidsequenz A, zumindest abschnittsweise, insbesondere zumindest zu dem endständigen 5'-Abschnitt, komplementäre Polynucleotidsequenz, insbesondere DNA-Sequenz auf, im Folgenden auch als Primernucleotidsequenz cA bezeichnet, und darüber hinaus cZIP-Sequenzabschnitte, die für die nachfolgende Hybridisierung und Identifizierung Verwendung finden. Diese Primer werden im Folgenden als Primer-cZIP X-Polynucleotide und Primer-cZIP Y-Polynucleotide bezeichnet.

In einer Ausführungsform ist bevorzugt vorgesehen, dass die zu der Polynucleotidsequenz A komplementäre Primernucleotidsequenz cA unmittelbar und daher ohne zwischenliegende Nucleotide an den cZIP-Sequenzabschnitt angrenzt. In einer weiteren Ausführungsform kann vorgesehen sein, dass zwischen der zu der Polynucleotidsequenz A komplementären Primernucleotidsequenz cA und dem cZIP-Sequenzabschnitt ein oder mehrere Nucleotide, insbesondere Polynucleotidsequenzabschnitte, angeordnet sind.

Das Primer-cZIP X-Polynucleotid weist eine Primernucleotidsequenz cA und eine cZIP X-Nucleotidsequenz auf, insbesondere besteht das Primer-cZIP X-Polynucleotid aus der Primernucleotidsequenz cA und der cZIP X-Nucleotidsequenz. Die Primernucleotidsequenz cA ist komplementär zu dem 5'-endständigen Abschnitt der Polynucleotidsequenz A, vorzugsweise zu zumindest dem 5'-endständigen Abschnitt der Polynucleotidsequenz A, vorzugsweise zu der Polynucleotidsequenz A. Die cZIP X-Nucleotidsequenz ist komplementär zu dem ersten Gegen-ZIP des Nucleotid-Mikroarrays.

Das Primer-cZIP Y-Polynucleotid weist die Primernucleotidsequenz cA und eine cZIP Y-Nucleotidsequenz auf, insbesondere besteht das Primer-cZIP Y-Polynucleotid aus der Primernucleotidsequenz cA und der cZIP Y-Nucleotidsequenz. Die cZIP Y-Nucleotidsequenz ist komplementär zu dem zweiten Gegen-ZIP des Nucleotid-Mikroarrays.

In einer besonders bevorzugten Ausführungsform der vorliegenden Erfindung befindet sich die cZIP X- und cZIP Y-Nucleotidsequenz 5'-wärts zur Primernucleotidsequenz cA. In einer bevorzugten Ausführungsform der vorliegenden Erfindung ist die cZIP X- und cZIP Y-Nucleotidsequenz an das 5'-Ende der Primernucleotidsequenz cA chemisch gebunden.

In einer bevorzugten Ausführungsform ist die cZIP X-Nucleotidsequenz eine DNA-cZIP X-Nucleotidsequenz, insbesondere eine D-DNA-cZIP X-Nucleotidsequenz.

In einer bevorzugten Ausführungsform der vorliegenden Erfindung ist die cZIP Y-Nucleotidsequenz eine DNA-cZIP Y-Nucleotidsequenz, insbesondere eine D-DNA-cZIP Y-Nucleotidsequenz.

Erfindungsgemäß bevorzugt ist die Primernucleotidsequenz cA so lang, dass eine Sequenz-spezifische Bindung, das heißt Sequenzspezifische Hybridisierung, der Primer-Nucleotidsequenz cA an den endständigen 5'-Abschnitt der Polynucleotidsequenz A erfolgen kann. Die Hybridisierung oder Bindung bleibt bevorzugt zumindest über die Dauer der erfindungsgemäßen Verfahrenschritte f) bis i) erhalten.

In einer bevorzugten Ausführungsform weist die Primernucleotidsequenz cA mindestens fünf Nucleotide, bevorzugt mindestens acht Nucleotide, bevorzugt mindestens 12 Nucleotide, bevorzugt mindestens 15 Nucleotide, bevorzugt mindestens 18 Nucleotide, bevorzugt mindestens 20 Nucleotide, bevorzugt 5 bis 50 Nucleotide, bevorzugt 10 bis 40 Nucleotide, bevorzugt 15 bis 30 Nucleotide, bevorzugt 18 bis 25 Nucleotide, bevorzugt 20 bis 22 Nucleotide auf.

In einer bevorzugten Ausführungsform der vorliegenden Erfindung weist der endständige 5'-Abschnitt der Polynucleotidsequenz A eine Länge, das heißt eine Anzahl von miteinander verbundenen Nucleotiden auf, die ausreicht eine sequenzspezifische Bindung der Primernucleotidsequenz cA zu ermöglichen, insbesondere mindestens 5 Nucleotide, bevorzugt mindestens 8 Nucleotide, bevorzugt mindestens 12 Nucleotide, bevorzugt mindestens 15 Nucleotide, bevorzugt mindestens 18 Nucleotide, bevorzugt mindestens 20 Nucleotide, bevorzugt 5 bis 50 Nucleotide, bevorzugt 10 bis 40 Nucleotide, bevorzugt 15 bis 30 Nucleotide, bevorzugt 18 bis 25 Nucleotide, bevorzugt 20 bis 22 Nucleotide, auf.

In einer bevorzugten Ausführungsform werden die Sequenzinformationen für die Herstellung der in Schritt b) bereitgestellten Primer-cZIP X- und Primer-cZIP Y-Polynucleotide, insbesondere für die konkret einzusetzende Primernucleotidsequenz cA, aus verfügbaren Gendatenbanken extrahiert, zum Beispiel aus miRBase oder vergleichbaren Datenbanken, und die revers komplementäre Nucleotidsequenz dazu, also eine DNA-Sequenz, hergestellt und an 5'-Ende mit einer, vorzugsweise DNA-, vorzugsweise D-DNA cZIP-Sequenz verbunden.

In einem Schritt c) wird ein Nucleotid-Mikroarray bereitgestellt, umfassend einen Träger und zumindest zwei Gegen-ZIPs, wobei auf den Nucleotid-Mikroarrayträger an unterschiedlichen Positionen mindestens zwei unterschiedliche erste und zweite Gegen-ZIPs immobilisiert sind.

In einer bevorzugten Ausführungsform weisen die auf dem Mikroarrayträger vorhandenen Gegen-ZIPs keine Homologie und/oder Kreuzhybridisierung zu irgendwelchen Nucleotidsequenzen von Organismen, insbesondere dem Menschen, auf. Sie sind bevorzugt universell einsetzbar und können bevorzugt zur parallelen, das heißt zeitgleichen, Detektion unterschiedlicher Nukleinsäuren, insbesondere DNA und/oder mRNA, eingesetzt werden.

In einer bevorzugten Ausführungsform sind die ersten und zweiten auf dem Nucleotid-Mikroarrayträger an unterschiedlichen Positionen immobilisierten Gegen-ZIPs Oligonucleotide mit einer Länge von 10 bis 50 Nucleotiden, bevorzugt 15 bis 40 Nucleotiden, bevorzugt 18 bis 35 Nucleotiden, bevorzugt 20 bis 30 Nucleotiden, bevorzugt 22 bis 26 Nucleotiden, bevorzugt 24 Nucleotiden. Die ersten und die zweiten Gegen-ZIPs unterscheiden sich voneinander in mindestens 6, bevorzugt mindestens 8, bevorzugt mindestens 9, bevorzugt mindestens 10 Nucleotiden. Die ersten und die zweiten Gegen-ZIPs sind bevorzugt weder komplementär noch palindromisch zueinander.

Die ersten und die zweiten Gegen-ZIPs sind bevorzugt unmittelbar auf dem Nucleotid-Mikroarrayträger an den unterschiedlichen Positionen immobilisiert. Bevorzugt sind diese Gegen-ZIPs über einen Spacer, bevorzugt einen Nucleotidspacer, auf dem Nucleotid-Mikroarrayträger immobilisiert.

Die Immobilisierung der ersten und der zweiten Gegen-ZIPs ist bevorzugt durch eine chemische Bindung realisiert. In einer bevorzugten Ausführungsform weist der Nucleotid-Mikroarrayträger chemische funktionale Gruppen auf, die mit dem einen Ende, bevorzugt mit dem 3'-Ende der ersten und/oder zweiten Gegen-ZIPs reagieren können. Der Träger ist bevorzugt ein Glasträger und/oder ein Plastikträger. Bevorzugt ist der Nucleotid-Mikroarrayträger mit einer endständige Amino- und/oder Epoxidgruppen aufweisenden Schicht beschichtet. In einer bevorzugten Ausführungsform weisen die Spacer, bevorzugt die Gegen-ZIPs, chemisch funktionelle Gruppen auf, die mit den Amino- und/oder Epoxidgruppen des Nucleotid-Arrayträgers reagieren können.

In einer bevorzugten Ausführungsform sind die ersten Gegen-ZIPs in anderen Bereichen auf den Nucleotid-Mikroarrayträger immobilisiert als die zweiten Gegen-ZIPs. Bevorzugt weist der Nucleotid-Mikroarrayträger mindestens zwei Bereiche auf, bevorzugt mindestens drei, bevorzugt mindestens vier, bevorzugt mindestens fünf, bevorzugt mindestens 6, bevorzugt mindestens acht Bereiche auf, wobei in mindestens einem Bereich, bevorzugt mindestens zwei Bereiche, bevorzugt mindestens drei Bereiche, bevorzugt mindestens vier Bereiche ausschließlich die ersten Gegen-ZIPs immobilisiert sind und wobei in mindestens einem Bereich, bevorzugt in mindestens zwei Bereichen, bevorzugt in mindestens drei Bereichen, bevorzugt in mindestens vier Bereichen ausschließlich die zweiten Gegen-ZIPs immobilisiert sind.

In einer bevorzugten Ausführungsform sind die Bereiche, in denen die ersten Gegen-ZIPs immobilisiert sind, zu dem Bereich, in dem die zweiten Gegen-ZIPs immobilisiert sind, etwa gleich groß, insbesondere gleich groß. Besonders bevorzugt sind die ersten Gegen-ZIPs auf dem Nucleotid-Mikroarrayträger auf der einen Seite und die zweiten Gegen-ZIPs auf der anderen Seite des Nucleotid-Mikroarrayträgers immobilisiert.

In erfindungsgemäß bevorzugter Ausführungsform können die Verfahrensschritte, im Folgenden auch als Schritte bezeichnet, a), b) und c) gleichzeitig oder in beliebiger Reihenfolgen aufeinander folgend durchgeführt werden.

In Schritt d) des erfindungsgemäßen Verfahrens werden die in der Probenlösung vorhandenen ersten und zweiten RNA-Moleküle zumindest zum Teil, bevorzugt mindestens 90 %, bevorzugt 91 %, bevorzugt 92 %, bevorzugt 93 %, bevorzugt 94 %, bevorzugt 95 %, bevorzugt 96 %, bevorzugt 97 %, bevorzugt 98 %, bevorzugt 99 % aller RNA-Moleküle, bevorzugt alle RNA-Moleküle adenyliert, bevorzugt polyadenyliert.

In einer bevorzugten Ausführungsform werden mindestens 50 %, bevorzugt mindestens 60 %, bevorzugt mindestens 70 %, bevorzugt mindestens 80 %, bevorzugt mindestens 90 %, bevorzugt mindestens 91 %, bevorzugt mindestens 92 %, bevorzugt mindestens 93 %, bevorzugt mindestens 94 %, bevorzugt mindestens 95 %, bevorzugt mindestens 96 %, bevorzugt mindestens 97 %, bevorzugt mindestens 98 %, bevorzugt mindestens 99 % aller ersten RNA-Moleküle, bevorzugt alle ersten RNA-Moleküle und mindestens 50 %, bevorzugt mindestens 60 %, bevorzugt mindestens 70 %, bevorzugt mindestens 80 %, bevorzugt mindestens 90 %, bevorzugt mindestens 91 %, bevorzugt mindestens 92 %, bevorzugt mindestens 93 %, bevorzugt mindestens 94 %, bevorzugt mindestens 95 %, bevorzugt mindestens 96 %, bevorzugt mindestens 97 %, bevorzugt mindestens 98 %, bevorzugt mindestens 99 % aller zweiten RNA-Moleküle, bevorzugt alle zweiten RNA-Moleküle in der Probenlösung adenyliert, bevorzugt polyadenyliert.

Bei der in Schritt d) durchführten Adenylierung werden das 5'-Ende oder beide Enden, nämlich das 3'-Ende und das 5'-Ende, der in der Lösung vorhandenen ersten und zweiten RNA-Moleküle adenyliert, bevorzugt polyadenyliert. Bevorzugt werden im Mittel, das heißt im Durchschnitt, am 3'-Ende und/oder 5'-Ende eines jeden ersten und zweiten RNA-Moleküls mindestens ein Adenin, bevorzugt mindestens zwei Adenine, bevorzugt mindestens fünf Adenine, bevorzugt mindestens zehn Adenine in die Polynukleodidsequenz eingebaut.

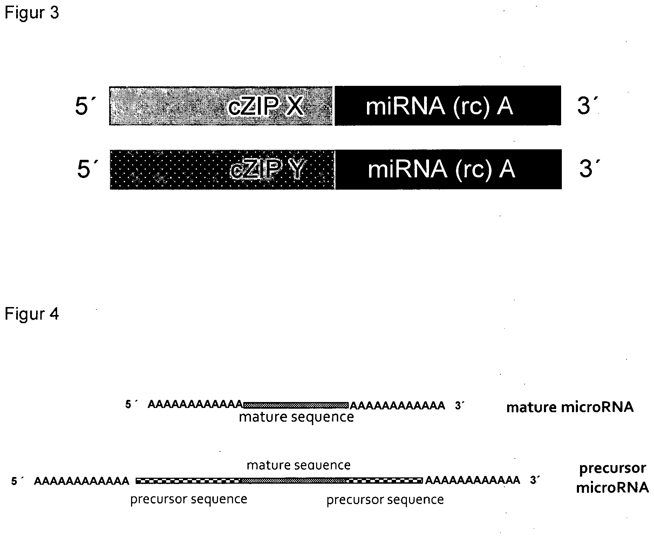

In bevorzugter Ausführungsform können die in Schritt a) bereitgestellten ersten und zweiten RNA-Moleküle vor oder während der Polyadenylierung gemäß Schritt d) amplifiziert werden, beispielsweise mittels T7-basierter linearer Amplifikationsverfahren, insbesondere mit einem "NCode" miRNA Amplification System von Invitrogen, Darmstadt, Deutschland (Mattie et al., Mol. Cancer, 2006 (5), 24). Vorzugsweise werden die bereitgestellten RNA-Moleküle während der Adenylierung amplifiziert, insbesondere wie folgt:
In einer bevorzugten Ausführungsform zur Adenylierung am 5'- und 3'-Ende der ersten und zweiten RNA-Moleküle wird in dem Schritt d) die erste und die zweite RNA, insbesondere mikroRNA, zunächst am 3'-Ende polyadenyliert mittels einer Poly(A)Polymerase. Anschließend findet bevorzugt eine Hybridisierung des polyadenylierten Endes mit einem reversen Transkriptions-Oligo dT Primer statt. In einem darauffolgenden Schritt wird bevorzugt, beginnend am 3'-Ende des reversen Transkriptions-Oligo dT Primers, eine reverse Transkription mittels einer reversen Transkriptase durchgeführt, wodurch eine cDNA-Kopie der ersten und zweiten mikroRNA erhalten wird. Danach wird bevorzugt die erste und zweite mikroRNA abgebaut. Bevorzugt wird die erhaltene cDNA-Kopie der ersten und zweiten mikroRNA aufgereinigt. Anschließend wird bevorzugt am 3'-Ende der cDNA-Kopie der ersten und zweiten mikroRNA eine Strangverlängerung durchgeführt, die allein desoxy-Thymidintriphosphat am 5'-Ende einbaut unter Verwendung einer terminalen Deoxynucleotidtransferase. Bevorzugt wird anschließend ein oligomeres T7-Template am 3'-Ende der cDNA eingebaut. Dann wird bevorzugt das 3'-Ende des einsträngigen cDNA-Stranges mit Nucleotiden unter Verwendung eines Klenow-Enzyms aufgefüllt, um so einen doppelsträngigen T7-Promoter herzustellen. Das T7-Template enthält einen Blocker, der die zweite Strangsynthese in 3'-Richtung verhindert. Anschließend wird bevorzugt eine Transkription mit einer T7-RNA-Polymerase durchgeführt. Danach wird bevorzugt die cDNA-Kopie der ersten und zweiten mikroRNA abgebaut und bevorzugt eine erste und zweite mikroRNA erhalten, die auf beiden Seiten, das heißt am 3'-Ende und am 5'-Ende polyadenyliert ist (siehe Mattie et al., Molecular Cancer, 2006, 5(24), Figur 3).

In einer bevorzugten Ausführungsform zur Adenylierung am 5'-Ende der ersten und zweiten RNA-Moleküle wird die am 5'-Ende polyadenylierte erste und zweite RNA dadurch bereitgestellt, dass die zu den ersten und zweiten RNA-Molekülen revers komplementäre DNA-Sequenz am 3'-Ende mit einer poly-dT₁₄-Sequenz und daran anschließend mit einer revers komplementären T7-RNA-Polymerase-Promotersequenz verbunden wird. In einem darauffolgenden Schritt wird bevorzugt zunächst der T7-Promoter vervollständigt. Anschließend findet bevorzugt eine Amplifikation durch In-Vitro-Transkription der T7-cDNA statt. Dazu wird bevorzugt diese T7-cDNA mit einer T7-RNA-Polymerase versetzt und zusätzlich bevorzugt mit einem Ribonuklease-Inhibitor, insbesondere RNaseOut. Anschließend wirddie am 5'-Ende polyadenylierte erste und zweite RNA von der cDNA abgetrennt, insbesondere aufgereinigt, z. B. über eine chromatographische Säule.

In einer bevorzugten Ausführungsform der vorliegenden Erfindung wird zunächst eine Polyadenylierung gemäß Schritt d) durchgeführt und anschließend eine Amplifikation der polyadenylierten ersten und zweiten RNA-Moleküle durchgeführt.

In einer bevorzugten Ausführungsform der vorliegenden Erfindung ist die Primernucleotidsequenz cA komplementär zu dem endständigen 5'-Abschnitt der Polynucleotidsequenz A, die cZIP X- und die cZIP Y-Nucleotidsequenz befindet sich 5'-wärts zu der Primernucleotidsequenz cA in dem Primer-cZIP-Polynucleotid, das zweite RNA-Molekül weist das Nucleotidelement P 5'-wärts zu der Polynucleotidsequenz A auf und das 5'-Ende zumindest des ersten RNA-Moleküls wird in Schritt d) adenyliert, so dass in bevorzugter Ausführungsform eine komplementäre fluoreszenzmarkierende Strangverlängerung in 3'-Richtung von der Primernucleotidsequenz cA aus gesehen stattfindet.

In einer bevorzugten Ausführungsform der vorliegenden Erfindung muss die Primernucleotidsequenz cA komplementär zu dem endständigen 5'-Abschnitt der Polynucleotidsequenz A sein, die cZIP X- und die cZIP Y-Nucleotidsequenz befindet sich 5'-wärts zu der Primernucleotidsequenz cA in dem Primer-cZIP-Polynucleotid und das zweite RNA-Molekül muss das Nucleotidelement P 5'-wärts zu der Polynucleotidsequenz A aufweisen, wenn in Schritt d) zumindest das erste RNA-Molekül zumindest am 5'-Ende adenyliert wird.

In einer bevorzugten Ausführungsform der vorliegenden Erfindung ist die Primernucleotidsequenz cA komplementär zu dem endständigen 5'-Abschnitt der Polynucleotidsequenz A, wenn das 3'-Ende und das 5'-Ende des ersten RNA-Moleküls adenyliert wird und das zweite RNA-Molekül das Nucleotidelement P 3'-wärts und 5'-wärts zu der Polynucleotidsequenz A aufweist, wobei das Nucleotidelement P 3'-wärts und 5'-wärts gleich oder verschieden ist.

In einer bevorzugten Ausführungsform findet die in Schritt d) durchgeführte Adenylierung, insbesondere Polyadenylierung, am 5'-Ende, bevorzugt am 3'-Ende und am 5'-Ende des ersten und des zweiten RNA-Moleküls statt. In einer bevorzugten Ausführungsform der vorliegenden Erfindung findet an beiden Seiten des Polynucleotidstranges des ersten und des zweiten RNA-Moleküls eine Polyadenylierung statt.

Die aus Verfahrensschritt d) erhaltene Lösung, die die ersten und zweiten adenylierten, insbesondere polyadenylierten, RNA-Moleküle aufweist, wird in Schritt e) in eine erste Lösung und eine zweite Lösung aufgeteilt.

In einer bevorzugten Ausführungsform ist das Verhältnis der Volumina von der ersten Lösung zu der zweiten Lösung 3:1 bis 1:3, bevorzugt 2:1 bis 1:2, bevorzugt 1,5:1 bis 1:1,5, bevorzugt 1:1.

In Schritt f) wird eine Hybridisierung des Primer cZIP X-Polynucleotids mit dem ersten und zweiten adenylierten RNA-Molekül, enthaltend in der ersten Lösung aus Schritt e), durchgeführt.

In Schritt g) wird eine Hybridisierung des Primer cZIP Y-Polynucleotids mit dem ersten und zweiten adenylierten RNA-Molekül, enthaltend in der zweiten Lösung aus Schritt e), durchgeführt.

In beiden Schritten f) und g) werden für eine Hybridisierung der Primer-cZIP X- und -cZIP Y-Polynucleotide mit den adenylierten, vorzugsweise adenylierten und amplifizierten, RNA-Molekülen geeignete Bedingungen, insbesondere geeignete Salzkonzentrationen, pH-Werte und Temperaturen eingestellt.

Die Schritte f) und g) werden demgemäß unter Bedingungen durchgeführt, die eine Hybridisierung der Primernucleotidsequenz cA mit dem komplementären endständigen 5'-Abschnitt der Polynucleotidsequenz A ermöglichen.

In den Schritten f) und g) lagert sich die Primernucleotidsequenz cA an den komplementären endständigen 5'-Abschnitt der Polynucleotidsequenz A des mindestens ersten und zweiten RNA-Moleküls an.

Die Schritte f) und g) werden erfindungsgemäß in unterschiedlichen Behältnissen durchgeführt, da die ersten und zweiten adenylierten RNA-Moleküle einerseits dem Primer-cZIP X-Polynucleotid und getrennt davon dem Primer-cZIP Y-Polynucleotid ausgesetzt werden sollen. Erfindungsgemäß ist es möglich, die Verfahrensschritte f) und g) gleichzeitig oder aufeinanderfolgend durchzuführen.

In Schritt h) wird zu der Lösung A1 ein mit einem Fluoreszenzfarbstoff markiertes erstes Nucleosidtriphosphat, insbesondere desoxy-Uridintriphosphat oder desoxy-Thymidinphosphat, gegeben.

In einer bevorzugten Ausführungsform der vorliegenden Erfindung wird in Schritt h) die in Schritt f) erhaltene Lösung A1 zusätzlich mit mindestens einem nicht fluoreszenzmarkierten Nucleosidtriphosphat ausgewählt aus desoxy-Thymidintriphosphat und desoxy-Uridintriphosphat versetzt. Bevorzugt wird die in Schritt f) erhaltene Lösung A1 in Schritt h) mit einem nicht fluoreszenzmarkierten desoxy-Thymidintriphosphat versetzt, wenn das desoxy-Uridintriphosphat fluoreszenzmarkiert ist. Bevorzugt wird die in Schritt f) erhaltene Lösung A1 in Schritt h) mit einem nicht fluoreszenzmarkierten desoxy-Uridintriphosphat versetzt, wenn das desoxy-Thymidintriphosphat fluoreszenzmarkiert ist.

Bevorzugt wird die in Schritt f) erhaltene Lösung A1 in Schritt h) mit einem nicht fluoreszenzmarkierten desoxy-Thymidintriphosphat und einem fluoreszenzmarkierten desoxy-Uridintriphosphat versetzt. Bevorzugt wird die in Schritt f) erhaltene Lösung A1 in Schritt h) mit einem nicht fluoreszenzmarkierten desoxy-Uridintriphosphat und einem fluoreszenzmarkierten desoxy-Thymidintriphosphat versetzt.

In einer bevorzugten Ausführungsform erfolgt die enzymatische Strangverlängerung in Schritt h) ausschließlich mit dem mindestens einen ersten Nucleosidtriphosphat, das mit einem Fluoreszenzfarbstoff markiert ist, wobei das erste Nucleosidphosphat ausgewählt ist aus desoxy-Thymidintriphosphat und desoxy-Uridintriphosphat, und mit unmarkierten desoxy-Thymidintriphosphat und/oder unmarkierten desoxy-Uridintriphosphat.

Der Schritt h) wird unter Bedingungen und mit Hilfe mindestens eines Enzyms, insbesondere einer Polymerase, durchgeführt, die einen Anbau des markierten ersten Nucleosidtriphosphats an das 3'-Ende der Primernucleotidsequenz cA des Primer-cZIP X-Polynucleotids, also eine komplementäre Strangverlängerung, erlauben. Ein Einbau des ersten Nucleosidtriphosphats erfolgt, wenn die gegenüberliegende, das heißt komplementäre Base des RNA-Moleküls ein Adenin ist. Dies trifft also dann zu, wenn an die Polynucleotidsequenz A des RNA-Moleküls kein Nucleotidelement P angrenzt, sondern vielmehr der Poly(A)-Abschnitt vorliegt, welcher in Schritt d) an das erste RNA-Molekül, insbesondere an das reife RNA-Molekül, angebracht wurde.

In einer Ausführungsform der vorliegenden Erfindung kann in der in Schritt a) bereitgestellten Probenlösung ein zweites RNA-Molekül mit einem Nucleotidelement P vorliegen, welches mit einem oder mehreren A (Adenin-Basen) an die Polynucleotidsequenz A angrenzt.

In einer derartigen Ausführungsform weist die Primernucleotidsequenz cA an ihrem 3'-Ende bevorzugt zusätzlich eine oder mehrere Uracil- und/oder Thymin-Basen auf. Diese Primernucleotidsequenz wird als Primernucleotidsequenz cAe bezeichnet. In dieser speziellen Ausführungsform ist die Primernucleotidsequenz cAe bevorzugt komplementär zu mindestens einem endständigen 5'-Abschnitt der Polynucleotidsequenz A und der mindestens einen Adenin-Base des zweiten RNA-Moleküls, welche unmittelbar an die Polynucleotidsequenz A angrenzt. Durch die Hybridisierung dieses zweiten RNA-Moleküls mit einem die Primernucleotidsequenz cAe aufweisenden Primer-cZIP X-Polynucleotid erfolgt bei der Strangverlängerung gemäß Schritt h) bevorzugt kein Anbau eines mit einem Fluoreszenzstoff markierten ersten Nucleosidtriphosphats 3'-wärts zu der Primernucleotidsequenz cAe. Die mindestens eine Adenin-Base, die unmittelbar an die Polynucleotidsequenz A des zweiten RNA-Moleküls angrenzt, liegt in dieser Ausführungsform bevorzugt nach der Hybridisierung gemäß Schritt f) hybridisiert mit den am 3'-Ende der Primernucleotidsequenz cAe vorhandenen Uracil- und/oder Thymin-Basen vor.

In einer derartigen Ausführungsform kann vorgesehen sein, den erfindungsgemäßen Nachweis des ersten und zweiten RNA-Moleküls, insbesondere der An- oder Abwesenheit des Nucleotidelements P, im zweiten RNA-Molekül mittels eines zusätzlichen zweiten markierten, insbesondere Cy-markierten, desoxy-Nucleotids, insbesondere dGTP bei Anwesenheit unmarkierter dNTP, durchzuführen.

Erfindungsgemäß wird dies insbesondere dadurch möglich, dass die verwendeten Vorläufer(precursor)-mikro-RNA-Nucleotidsequenzen bekannt sind und in gleicher Weise eine Fluoreszenzmarkierung analog der Vorläufer-Nucleotidsequenz erfolgen kann.

In einer derartigen Ausführungsform, also einer solchen, in der ein zweites RNA-Molekül mit einem Nucleotidelement P vorliegt, welches mit einem oder mehreren A an die Polynucleotidsequenz A angrenzt, erfolgt erfindungsgemäß bevorzugt eine Unterscheidung des ersten und zweiten RNA-Moleküls, das heißt der Nachweis der An- oder Abwesenheit des Nucleotidelements P, durch eine Quantifizierung des in Schritt k) detektierten Fluoreszenzsignals, das heißt der Fluoreszenzintensität der an den Mikroarray gebundenen markierten Primer-cZIP X- und Primer-cZIP Y-Polynucleotide.

In einer derartigen Ausführungsform kann es besonders bevorzugt vorgesehen sein, die Verfahrensschritte a) bis k) durchzuführen, wobei in Schritt e) die Lösung aus Schritt d) in drei Lösungen, insbesondere mit gleichen Volumina, geteilt, die erste und zweite Lösung gemäß der Schritte f) bis k) weiter behandelt und die dritte Lösung mit dem in Schritt b) bereitgestellten Primer-cZIP Y-Polynucleotid versetzt und eine Hybridisierung mit dem ersten und zweiten adenylierten RNA-Molekül unter Erhalt einer Lösung C1 durchgeführt wird, in einem weiteren Schritt die erhaltene Lösung C1 mit einem Nucleosidtriphosphat, das mit einem Fluoreszenzfarbstoff markiert ist, insbesondere desoxy-Guanosintriphosphat, und mit mindestens einem nicht mit einem Fluoreszenzfarbstoff markierten desoxy-Nucleosidtriphosphat ausgewählt aus der Gruppe bestehend aus desoxy-Adenosintriphosphat, desoxy-Cytidintriphosphat, desoxy-Thymidintriphosphat und desoxy-Uridintriphosphat versetzt und eine enzymatische Strangverlängerung des hybridisierten Primer-cZIP Y-Polynucleotids unter Erhalt einer Lösung C2 durchgeführt wird, wobei in einem weiteren Schritt die erhaltene Lösung C2 mit einen erste und zweite Gegen-ZIPs umfassenden Nucleotid-Mikroarray in Kontakt gebracht und eine Hybridisierung des Primer-cZIP Y-Polynucleotids mit den zweiten Gegen-ZIPs durchgeführt wird und wobei in einem weiteren Schritt ein differenziertes Nachweisen der mindestens zwei unterschiedlichen ersten und zweiten RNA-Moleküle durch eine Fluoreszenzdetektion erfolgt, insbesondere im quantitativen Vergleich zu der detektierten Fluoreszenz der Lösungen A2 und B2.

Der Schritt h) wird insbesondere unter Bedingungen durchgeführt, die einen Anbau des erstes Nucleosidtriphosphats an das 3'-Ende des Primer-cZIP X-Polynucleotids erlauben.

Der Anbau des markierten desoxy-Uridintriphosphats und/oder desoxy-Thymidintriphosphats findet bevorzugt allein an dem ersten RNA-Molekül statt. Erfindungsgemäß lagert sich die Primernucleotidsequenz cA an das erste RNA-Molekül so an, dass allein die durch die Adenylierung, insbesondere Polyadenylierung, eingeführten Adeninbasen 5'-wärts des hybridisierenden komplementären Bereichs der Polynucleotidsequenz A einsträngig vorliegen. Das markierte desoxy-Uridintriphosphat oder desoxy-Thymidintriphosphat wird, komplementär und strangverlängernd 3'-wärts zu dem an den komplementären Abschnitt des adenylierten, insbesondere polyadenylierten ersten RNA-Moleküls hybridisierten Primer-cZIP X- oder Y-Polynucleotid eingebaut, wodurch bevorzugt durch Umschreibung eine fluoreszenzmarkierte cDNA erhalten wird.

In einer besonders bevorzugten Ausführungsform ist vorgesehen, dass nachdem das Primer-cZIP X- oder cZIP Y-Polynucleotid an einen endständigen 5'-Abschnitt der Polynucleotidsequenz A hybridisiert hat, eine komplementäre Strangverlängerung in 3'-Richtung des hybridisierten Primer-cZIP X- oder Y-Polynucleotids durch eine zu dieser Funktion fähige Polymerase erfolgt.

In einem Schritt i) wird zu der in Verfahrensschritt g) erhaltenen Lösung B1 mindestens ein zweites Nucleosidtriphosphat, das mit einem Fluoreszenzfarbstoff markiert ist, gegeben, wobei das zweites Nuleosidtriphosphat ausgewählt ist aus der Gruppe bestehend aus desoxy-Guanosintriphosphat, desoxy-Adeninosintriphosphat und desoxy-Cytidintriphosphat, wodurch bevorzugt durch Umschreibung eine fluoreszenzmarkierte cDNA erhalten wird.

Die Lösung B1 enthält bevorzugt zusätzlich zu den vorgenannten markierten zweiten Nucleotidphosphaten nicht mit einem Fluroreszenzfarbstoff markierte, also unmarkierte Nucleosidtriphosphate, die bevorzugt ausgewählt sind aus der Gruppe bestehend aus desoxy-Guanosintriphosphat, desoxy-Adeninosintriphosphat, desoxy-Thymidintriphosphat, desoxy-Uridintriphosphat und desoxy-Cytidintriphosphat. In besonders bevorzugter Ausführungsform sind die zusätzlich vorhandenen nicht-markierten zweiten Nucleotidtriphosphate desoxy-Thymidintriphosphat, desoxy-Uridintriphosphat oder beide. Bevorzugt sind die nicht mit einem Fluoreszenzfarbstoff markierten Nucleosidtriphosphate verschieden von den zweiten Nucleosidtriphosphaten.

In einer bevorzugten Ausführungsform erfolgt die enzymatische Strangverlängerung in Schritt i) ausschließlich durch das mindestens eine zweite Nucleosidtriphosphat, das mit einem Fluoreszenzfarbstoff markiert ist, wobei das zweite Nucleosidtriphosphat ausgewählt ist aus der Gruppe bestehend aus desoxy-Guanosintriphosphat, desoxy-Adenosintriphosphat und desoxy-Cytidintriphosphat, und mit mindestens einem, bevorzugt vier, unmarkierten Nucleosidtriphosphaten ausgewählt aus der Gruppe bestehend aus desoxy-Adenosintriphosphat, desoxy-Guanosintriphosphat, desoxy-Thymidintriphosphat und desoxy-Cytidintriphosphat. Bevorzugt wird zur enzymatischen Strangverlängerung zusätzlich oder alternativ zu dem unmarkierten Thymidintriphosphat ein unmarkiertes Uridintriphosphat verwendet.

Der Schritt i) wird unter Bedingungen und mit Hilfe mindestens eines Enzyms, insbesondere einer Polymerase, durchgeführt, die einen Anbau der zweiten markierten, und bevorzugt zusätzlich der nicht mit einem Fluoreszenzfarbstoff markierten, Nucleosidtriphosphate an das 3'-Ende der Primernucleotidsequenz cA des Primer-cZIP Y-Polynucleotids, also eine komplementäre Strangverlängerung, erlauben.

In einer bevorzugten Ausführungsform der vorliegenden Erfindung lagert sich in Schritt i) die Primernukleotidsequenz cA, entsprechend Schritt g), an die Polynukleotidsequenz A sowohl der ersten als auch der zweiten RNA-Moleküle an. In Schritt i) wird 3'-wärts zu der Primernukleotidsequenz cA der Strang komplementär verlängert. Bei dem ersten RNA-Molekül findet, sofern bevorzugt vorhanden, der Einbau des desoxy-Uridintriphosphat oder des desoxy-Thymidintriphosphat, das nicht mit einem Fluoreszenzfarbstoff markiert ist, statt. Bei dem zweiten RNA-Molekül findet, sofern bevorzugt vorhanden, der Einbau der nicht mit einem Fluoreszenzfarbstoff markierten Nucleosidtriphosphate, und in jedem Fall der mit einem Fluoreszenzfarbstoff markierten zweiten Nucleosidtriphosphate statt.

In dem Schritt h) werden demgemäß bevorzugt allein die ersten RNA-Moleküle fluoreszenzmarkiert. In dem Schritt i) werden bevorzugt allein die zweiten RNA-Moleküle fluoreszenzmarkiert.

Erfindungsgemäß bevorzugt ergibt sich daher, dass die zu dem ersten Gegen-ZIP komplementären Primer-cZIP X-Polynucleotide allein dann verlängert und damit fluoreszenzmarkiert sind, wenn ein erstes RNA-Molekül in der Probe vorhanden ist. Es ergibt sich auch, dass ein zu dem zweiten Gegen-ZIP komplementäres Primer-cZIP Y-Polynucleotid allein dann fluoreszenzmarkiert und strangverlängert ist, wenn ein zweites RNA-Molekül in der Probe vorhanden ist.

In einer bevorzugten Ausführungsform der vorliegenden Erfindung ist das erste und das zweite Nucleosidtriphosphat mit demselben Fluoreszenzfarbstoff markiert.

In einer bevorzugten Ausführungsform der vorliegenden Erfindung ist der Fluoreszenzfarbstoff ein Cyaninfarbstoff, insbesondere Cy3 oder Cy5, insbesondere Cy3.

In einer besonders bevorzugten Ausführungsform kann vorgesehen sein, dass die Schritte f) und h) in einem gemeinsamen Gefäß durchgeführt werden. In einer weiteren Ausführungsform kann vorgesehen sein, dass die Schritte g) und i) in einem gemeinsamen Gefäß durchgeführt werden. Erfindungsgemäß kann in einer weiteren bevorzugten Ausführungsform vorgesehen sein, dass jeweils die Verfahrensschritte f) und g) und/oder h) und i) jeweils gleichzeitig durchgeführt werden, wobei zumindest zwei unterschiedliche. Gefäße eingesetzt werden, nämlich eines, in dem die Lösung A1 und A2, und ein weiteres, in dem die Lösung B1 und B2 erhalten werden.

In einer bevorzugten Ausführungsform der vorliegenden Erfindung werden die in Schritte h) und i) erhaltenen und mit dem Primer-cZIP X-Polynucleotid oder Primer-cZIP Y-Polynucleotid hybridisierten und strangverlängerten, also markierten, ersten und zweiten RNAs bevorzugt durch einen Hitzeschritt denaturiert, oder bevorzugt werden die RNAs durch eine Ribonuklease, insbesondere Ribonuklease H abgebaut, bevor die strangverlängerten Primer-cZIP X-Polynucleotide und/oder Primer-cZIP Y-Polynucleotide mit dem in Schritt c) bereitgestellten Nucleotid-Mikroarray in Schritt j) in Kontakt gebracht werden.

In einem Verfahrensschritt j) werden die Lösungen A2 und B2 mit dem in Schritt c) bereitgestellten Nucleotid-Mikroarray unter für eine Hybridisierung geeigneten Bedingungen in Kontakt gebracht, sodass eine Hybridisierung der cZIP X-Polynucleotidsequenz und der cZIP Y-Polynucleotidsequenz mit den ersten und zweiten spezifischen Gegen-ZIPs erfolgt.

In einem bevorzugten optionalen Schritt werden die in den Schritten h) und i) erhaltenen Lösungen A2 und B2 vor Schritt j) zu einer Lösung C vereinigt. Diese Vereinigung findet bevorzugt durch einfaches Zusammenschütten statt.

In einer bevorzugten Ausführungsform der vorliegenden Erfindung wird nach dem Schritt j) und vor dem Schritt k) der Nucleotid-Mikroarray von allen nicht an den Gegen-ZIPs hybridisierten Nucleotidsequenzen befreit, insbesondere findet diese Befreiung durch einen Waschschritt statt.

In Schritt k) findet ein differenziertes Nachweisen der mindestens zwei unterschiedlichen ersten und zweiten RNA-Moleküle durch eine Fluoreszenzdetektion, insbesondere durch eine Fluoreszenzmessung, statt.

Diese Fluoreszenzdetektion dient der qualitativen oder quantitativen Bestimmung von am Nucleotid-Mikroarray gebundenen markierten Primer-cZIP X-/Y-Polynucleotide.

In einer besonders bevorzugten Ausführungsform kann die Fluoreszenzdetektion eine qualitative oder eine quantitative Fluoreszenzdetektion sein. Im Falle einer quantitativen Fluoreszenzdetektion findet der Nachweis der ersten und zweiten RNA-Moleküle durch Vergleich der Fluoreszenzintensität der markierten am Nucleotid-Mikroarray gebundenen Primer-cZIP X-/Y-Polynucleotide statt.

Da das Primer-cZIP X-Polynucleotid nur dann fluoreszenzmarkiert ist, wenn ein erstes RNA-Molekül in der Probenlösung vorlag und das Primer-cZIP Y-Polynucleotid nur dann fluoreszenzmarkiert ist, wenn ein zweites RNA-Molekül in der Probenlösung vorlag und darüber hinaus die ersten und zweiten Gegen-ZIPs räumlich getrennt voneinander auf dem Nucleotid-Mikroarray vorliegen, kann in Schritt k) ein differenzierter Nachweis der Anwesenheit des ersten und zweiten RNA-Moleküls in der Probenlösung erfolgen.

In bevorzugter Ausführungsform sind die Enzyme für Amplifikation und Polyadenylierung ausgewählt aus der Gruppe bestehend aus Poly A-Polymerase, Reverse Transkriptase, Ribonuklease-Inhibitor, Terminale Desoxynucleotidyl-Transferase, Klenow, T7 RNA Polymerase und Enzyme vergleichbarer Aktivität.

In bevorzugter Ausführungsform sind die Enzyme für cDNA-Synthese, Fluoreszenzmarkierung und Strangverlängerung ausgewählt aus der Gruppe bestehend aus Ribonuklease-Inhibitor, M-MuLV Reverse Transkriptase, Ribonuklease H und Enzyme vergleichbarer Aktivität.

In einer bevorzugten Ausführungsform wird die Amplifikation und/oder Strangverlängerung unter Anwesenheit und Wirkung eines Ribonuklease-Inhibitors, insbesondere RNaseOut durchgeführt.

In einer bevorzugten Ausführungsform der folgenden Erfindung wird die in Schritt f) erhaltene Lösung A1 und/oder die in Schritt g) erhaltene Lösung B1 mit einer reversen Transkriptase und/oder zusätzlich mit einem Ribonuklease-Inhibitor versetzt, gegebenenfalls sukzessive zueinander. In einer bevorzugten Ausführungsform kann im Anschluss an die mit der reversen Transkriptase erfolgte reverse Transkription eine Zugabe von RNAase H (Ribonuclease H) erfolgen.

In einer bevorzugten Ausführungsform der vorliegenden Erfindung weist die Probenlösung zusätzlich mindestens zwei unterschiedliche dritte und vierte RNA-Moleküle auf, wobei das dritte RNA-Molekül die Polynucleotidsequenz B ist und das vierte RNA-Molekül die Polynucleotidsequenz B und unmittelbar anschließend ein Nukleotidelement P1 aufweist.

In einer bevorzugten Ausführungsform der vorliegenden Erfindung weist die Probenlösung zusätzlich weitere RNA-Moleküle auf, wobei die einen RNA-Moleküle jeweils eine bestimmte Polynucleotidsequenz haben, die von den Polynucleotidsequenzen A, B und den anderen in der Probelösung vorkommenden Polynucleotidsequenzen verschieden ist, und die anderen RNA-Moleküle jeweils eine identische Polynucleotidsequenz zu einer der einen RNA-Moleküle und unmittelbar anschließend ein weiteres Nukleotidelement aufweisen.

In einer bevorzugten Ausführungsform der vorliegenden Erfindung wird ein Verfahren zum differenzierten Nachweis der mindestens zwei unterschiedlichen ersten und zweiten RNA-Moleküle, die jeweils eine identische Polynucleotidsequenz A aufweisen, und den dritten und vierten RNA-Moleküle, die jeweils eine identische Polynucleotidsequenz B aufweisen, durchgeführt mit den folgenden Schritten:
aa) Bereitstellen einer Probenlösung, die die mindestens vier unterschiedlichen ersten, zweiten, dritten und vierten RNA-Moleküle aufweist, wobei das erste RNA-Molekül die Polynucleotidsequenz A ist und das zweite RNA-Molekül die Polynucleotidsequenz A und unmittelbar anschließend 5'-wärts ein Nucleotidelement P aufweist, wobei das dritte RNA-Molekül die Polynucleotidsequenz B ist und das vierte RNA-Molekül die Polynucleotidsequenz B und unmittelbar anschließend 5'-wärts ein Nucleotidelement P1 aufweist,
bb) Bereitstellen eines eine Primernukleotidsequenz cA und eine cZIP X-Nukleotidsequenz aufweisenden Primer-cZIP X1-Polynucleotids, wobei die Primernukleotidsequenz cA komplementär zu einem endständigen 5'-Abschnitt der Polynucleotidsequenz A ist, eines die Primernukleotidsequenz cA und eine cZIP Y-Nukleotidsequenz aufweisenden Primer-cZIP Y1-Polynucleotids, eines eine Primernukleotidsequenz cB und eine cZIP X-Nukleotidsequenz aufweisenden Primer-cZIP X2-Polynucleotids, wobei die Primernukleotidsequenz cB komplementär zu einem endständigen 5'-Abschnitt der Polynucleotidsequenz B ist, und eines die Primernukleotidsequenz cB und eine cZIP Y-Nukleotidsequenz aufweisenden Primer-cZIP Y2-Polynucleotids,
cc) Bereitstellen eines Nucleotid-Microarrays umfassend mindestens vier unterschiedliche erste, zweite, dritte und vierte auf einem Nucleotid-Microarrayträger an unterschiedlichen Positionen immobilisierte Gegen-ZIPs, wobei das erste Gegen-ZIP komplementär zu der cZIP X1-Nukleotidsequenz, das zweite Gegen-ZIP komplementär zu der cZIP Y1-Nukleotidsequenz, das dritte Gegen-ZIP komplementär zu der cZIP X2-Nukleotidsequenz und das vierte Gegen-ZIP komplementär zu der cZIP Y2-Nukleotidsequenz ist,
dd) Adenylierung der in der Lösung aus Schritt a) vorhandenen ersten, zweiten, dritten und vierten RNA-Moleküle, wobei eine erste, zweite, dritte und vierte adenylierte RNA-Moleküle aufweisende Lösung erhalten wird,
ee) Teilen der die ersten, zweiten, dritten und vierten adenylierten RNA-Moleküle aufweisenden Lösung aus Schritt dd) in eine erste, zweite, dritte und vierte Lösung,
ff) Versetzen der ersten Lösung mit dem in Schritt bb) bereitgestellten Primer-cZIP X1-Polynucleotid und Durchführung einer Hybridisierung mit dem ersten und zweiten adenylierten RNA-Molekül, wobei eine Lösung A1 erhalten wird,
gg) Versetzen der zweiten Lösung mit dem in Schritt bb) bereitgestellten Primer cZIP Y1-Polynucleotid und Durchführung einer Hybridisierung mit dem ersten und zweiten adenylierten RNA-Molekül, wobei eine Lösung B1 erhalten wird,
hh) Versetzen der in Schritt ff) erhaltenen Lösung A1 mit mindestens einem ersten Nucleosidtriphosphat, das mit einem Fluoreszenzfarbstoff markiert ist, wobei das erste Nucleosidtriphosphat ausgewählt ist aus desoxy-Thymidintriphosphat und desoxy-Uridintriphosphat, und Durchführung einer enzymatischen Strangverlängerung des mit den ersten und zweiten adenylierten RNA-Molekülen hybridisierten Primer-cZIP X1-Polynukleotids, wobei eine Lösung A2 erhalten wird,
ii) Versetzen der in Schritt gg) erhaltenen Lösung B1 mit mindestens einem zweiten Nucleosidtriphosphat, das mit einem Fluoreszenzfarbstoff markiert ist, und wobei das zweite Nucleosidtriphosphat ausgewählt ist aus der Gruppe bestehend aus desoxy-Guanosintriphosphat, desoxy-Adenosintriphosphat und desoxy-Cytidintriphosphat sowie mit unmarkierten dNTPs (auch als desoxy-Nucleosidtriphosphat bezeichnet), und Durchführung einer enzymatischen Strangverlängerung des mit den ersten und zweiten adenylierten RNA-Molekülen hybridisierten Primer-cZIP Y1-Polynucleotids, wobei eine Lösung B2 erhalten wird,
jj) Inkontaktbringen der in den Schritten hh) und ii) erhaltenen Lösungen A2 und B2 mit dem in Schritt cc) bereitgestellten ersten Nucleotid-Mikroarray und Durchführung einer Hybridisierung der Primer-cZIP X1- und Primer-cZIP Y1-Polynucleotide mit den ersten und zweiten Gegen-ZIPs,
kk) differenziertes Nachweisen der mindestens zwei unterschiedlichen ersten und zweiten RNA-Moleküle durch eine Fluoreszenzdetektion,
II) Versetzen der dritten Lösung mit dem in Schritt bb) bereitgestellten Primer-cZIP X2-Polynucleotid und Durchführung einer Hybridisierung mit dem dritten und vierten adenylierten RNA-Molekül, wobei eine Lösung A3 erhalten wird,
mm) Versetzen der vierten Lösung mit dem in Schritt bb) bereitgestellten Primer cZIP Y2-Polynucleotid und Durchführung einer Hybridisierung mit dem dritten und vierten adenylierten RNA-Molekül, wobei eine Lösung B3 erhalten wird,
nn) Versetzen der in Schritt II) erhaltenen Lösung A3 mit mindestens einem ersten Nucleosidtriphosphat, das mit einem Fluoreszenzfarbstoff markiert ist, wobei das erste Nucleosidtriphosphat ausgewählt ist aus desoxy-Thymidintriphosphat und desoxy-Uridintriphosphat, und Durchführung einer enzymatischen Strangverlängerung des mit den dritten und vierten adenylierten RNA-Molekülen hybridisierten Primer-cZIP X2-Polynukleotids, wobei eine Lösung A4 erhalten wird,
oo) Versetzen der in Schritt mm) erhaltenen Lösung B3 mit mindestens einem zweiten Nucleosidtriphosphat, das mit einem Fluoreszenzfarbstoff markiert ist, und wobei das zweite Nucleosidtriphosphat ausgewählt ist aus der Gruppe bestehend aus desoxy-Guanosintriphosphat, desoxy-Adenosintriphosphat und desoxy-Cytidintriphosphat sowie mit unmarkierten dNTPs, und Durchführung einer enzymatischen Strangverlängerung des mit den dritten und vierten adenylierten RNA-Molekülen hybridisierten Primer-cZIP Y2-Polynucleotids, wobei eine Lösung B4 erhalten wird,
pp) Inkontaktbringen der in den Schritten nn) und oo) erhaltenen Lösungen A4 und B4 mit dem in Schritt cc) bereitgestellten Nucleotid-Mikroarray und Durchführung einer Hybridisierung der Primer-cZIP XZ- und Primer-cZIP YZ-Polynucleotide mit den dritten und vierten Gegen-ZIPs,
qq) differenziertes Nachweisen der mindestens zwei unterschiedlichen dritten und vierten RNA-Moleküle durch eine Fluoreszenzdetektion.

In einer bevorzugten Ausführungsform der vorliegenden Erfindung erfolgt die enzymatische Strangverlängerung in Schritt hh) ausschließlich mit dem mindestens einen ersten Nucleosidtriphosphat und unmarkierten Nucleosidtriphosphaten ausgewählt aus der Gruppe bestehend aus desoxy-Thymidintriphosphat und desoxy-Uridintriphosphat.

In einer bevorzugten Ausführungsform der vorliegenden Erfindung erfolgt die enzymatische Strangverlängerung in Schritt ii) ausschließlich mit dem mindestens einen zweiten Nucleosidtriphosphat und mit mindestens einem, bevorzugt vier unmarkierten Nucleosidtriphosphaten ausgewählt aus der Gruppe aus desoxy-Guanosintriphosphat, desoxy-Adenosintriphosphat, desoxy-Thymidintriphosphat und desoxy-Cytidintriphosphat. Bevorzugt wird zur enzymatischen Strangverlängerung zusätzlich oder alternativ zu dem unmarkierten Thymidintriphosphat ein unmarkiertes Uridintriphosphat verwendet.

In einer bevorzugten Ausführungsform können die Schritte ff) und II) gleichzeitig und gemeinsam in einem Gefäß durchgeführt werden.

In einer bevorzugten Ausführungsform können die Schritte gg) und mm) gleichzeitig und gemeinsam in einem Gefäß durchgeführt werden.

In einer bevorzugten Ausführungsform können die Schritte hh) und nn) gleichzeitig und gemeinsam in einem Gefäß durchgeführt werden.

In einer bevorzugten Ausführungsform können die Schritte ii) und oo) gleichzeitig und gemeinsam in einem Gefäß durchgeführt werden.

In einer bevorzugten Ausführungsform wird in Schritt ee) die Lösung aus Schritt dd) in eine erste und eine zweite Lösung aufgeteilt.

In einer bevorzugten Ausführungsform gelten die im Zusammenhang mit der Polynucleotidsequenz A getroffenen Aussagen ebenfalls für die Polynucleotidsequenz B, bevorzugt für alle weiteren Polynucleotidsequenzen.

In einer bevorzugten Ausführungsform gelten alle im Zusammenhang mit der Primernucleotidsequenz cA getroffenen Aussagen auch für die Primernucleotidsequenz cB, bevorzugt für alle weiteren Primernucleotidsequenzen.

In einer bevorzugten Ausführungsform gelten alle im Zusammenhang mit dem Polynucleotidelement P getroffenen Aussagen auch für das Polynucleotidelement P1, bevorzugt für alle weiteren Polynucleotidelemente.

In einer bevorzugten Ausführungsform gelten alle im Zusammenhang mit dem "Verfahren zum differenzierten Nachweis der mindestens zwei unterschiedlichen ersten und zweiten RNA-Moleküle" getroffenen Aussagen auch für das Verfahren zum differenzierten Nachweis der mindestens vier unterschiedlichen ersten und zweiten RNA-Moleküle, bevorzugt für alle weiteren RNA-Moleküle.

In einer bevorzugten Ausführungsform werden die Verfahrensschritte des erfindungsgemäßen Verfahrens, bevorzugt eines erfindungsgemäß bevorzugten Verfahrens, so angepasst, dass alle in einer Probenlösung vorhandenen RNAs, bevorzugt reifen und Vorläufer-mikroRNAs, bevorzugt einer humanen Zelle, bevorzugt von HeLa-Zellen, nachgewiesen werden können.

Erfindungsgemäß wird das technische Problem insbesondere durch ein Kit zur Durchführung eines Verfahrens zum differenzierten Nachweis von einer Probenlösung vorhandenen, mindestens zwei unterschiedlichen ersten und zweiten RNA-Molekülen, gelöst, die jeweils eine identische Polynucleotidsequenz A aufweisen. Dieser Kit enthält:
a1) einen Nucleotid-Mikroarray umfassend mindestens zwei unterschiedliche erste und zweite auf dem Nucleotid-Mikroarrayträger an unterschiedlichen Positionen immobilisierte Gegen-ZIPs,
b1) ein eine Primernukleotidsequenz cA und eine cZIP X-Nukleotidsequenz aufweisendes Primer-cZIP X-Polynucleotid, wobei die Primernukleotidsequenz cA komplementär zu einem endständigen Abschnitt der Polynucleotidsequenz A ist und wobei die cZIP X-Nukleotidsequenz komplementär zu dem ersten Gegen-ZIP des Nucleotid-Mikroarrays ist, und
c1) ein die Primernukleotidsequenz cA und eine cZIP Y-Nukleotidsequenz aufweisendes Primer-cZIP Y-Polynucleotid, wobei die cZIP Y-Nukleotidsequenz komplementär zu dem zweiten Gegen-ZIP des Nucleotid-Mikroarrays ist und
eine Lösung I, die mindestens ein mit einem Fluoreszenzfarbstoff markiertes erstes Nucleosidtriphosphat und eine Lösung II, die mindestens ein mit einem Fluoreszenzfarbstoff markiertes zweites Nucleosidtriphosphat enthält, und wobei das erste Nucleosidtriphosphat ausgewählt ist aus der Gruppe bestehend aus desoxy-Uridintriphosphat und desoxy-Thymidintriphosphat und wobei das zweite Nucleosidtriphosphat ausgewählt ist aus der Gruppe bestehend aus desoxy-Guanosintriphosphat, desoxy-Adenosintriphosphat, und desoxy-Cytidintriphosphat.

In besonders bevorzugter Ausführungsform weist der erfindungsgemäße Kit mindestens ein Enzym für eine Polyadenylierung und mindestens ein Enzym für eine Strangverlängerung auf.

In einer bevorzugten Ausführungsform weist der erfindungsgemäße Kit Instruktionen, insbesondere ein Instruktionsblatt, zur Durchführung des erfindungsgemäßen Verfahrens und/oder Instruktionen, insbesondere ein Instruktionsblatt, zur Anwendung, insbesondere auch diagnostischen oder therapeutischen Anwendung des Kits und Verfahrens auf.

Die Erfindung betrifft insbesondere eine Verwendung des erfindungsgemäßen Verfahrens und/oder des erfindungsgemäßen Kits in einem diagnostischen oder therapeutischen Verfahren, insbesondere in der medizinischen und/oder klinischen Diagnostik. Insbesondere kann dieses Verfahren und/oder Kit in der Tumorforschung eingesetzt werden, insbesondere für die verbesserte Klassifizierung, um insbesondere heterogene und aggressive, das heißt bösartige Tumore, insbesondere Lymphome, zu identifizieren.

Im Zusammenhang mit der vorliegenden Erfindung wird unter dem Begriff "ein erfindungsgemäßes Verfahren" insbesondere ein vorliegend beschriebenes Verfahren gemäß der Verfahrensabfolge der Schritt a) bis k) verstanden, wobei ein erfindungsgemäßes Verfahren auch eine oder mehrere der bevorzugten Ausgestaltungen dieser Verfahrenslehre ist.

Im Zusammenhang mit der vorliegenden Erfindung beziehen sich die Orientierungsangaben 5', 3', 5'-wärts und 3'-wärts immer, soweit nicht anders angegeben, auf eine relative Orientierung auf ein und demselben Strang eines RNA- oder DNA-Moleküls.

Weitere vorteilhafte Ausgestaltungen ergeben sich aus den Unteransprüchen.

Die Erfindung wird nachstehend ohne Beschränkung des allgemeinen Erfindungsgedankens anhand von Figuren, Tabellen und den dazugehörigen Ausführungsbeispielen beschrieben.

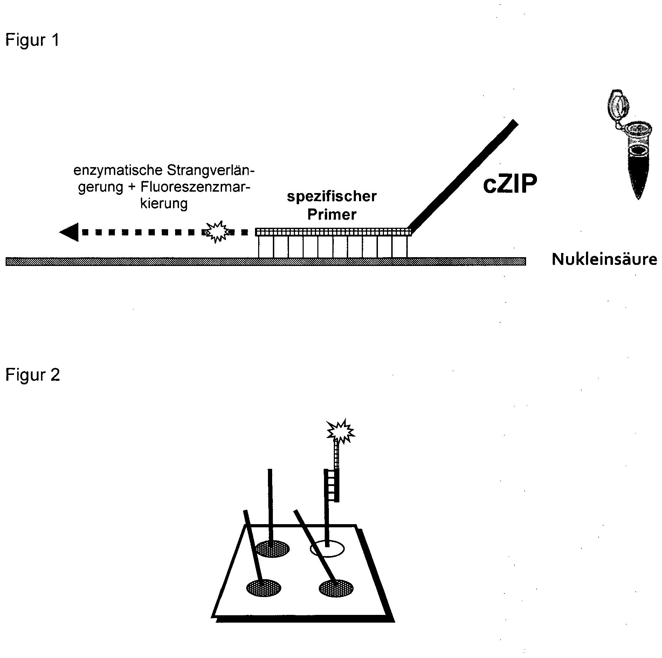

Figur 1 zeigt die Durchführung einer enzymatischen Reaktion zur Fluoreszenzmarkierung in Lösung unter Verwendung eines spezifizischen Primer-cZIP-Polynucleotids.
Figur 2 zeigt die spezifische Hybridisierung der cZIP-Polynucleotidsequenz mit ihrer komplementären Gegen-ZIP an einer definierten Position auf dem Nucleotid-Mikroarrayträger.
Figur 3 zeigt schematisch den Aufbau der mikroRNA spezifischen Primer-cZIP X- und Primer-cZIP Y-Polynucleotide aus jeweils einer cZIP-Sequenz und einer revers komplementären mikroRNA-spezifischen Primernucleotidsequenz cA (miRNA(rc)A).
Figur 4 zeigt polyadenylierte Polynucleotidsequenzen einer reifen und einer Vorläufer-mikroRNA. Die mikroRNA-Moleküle liegen nach Amplifikation der Methode von Mattie et al. jeweils am 3'- und am 5'-Ende polyadenyliert vor.

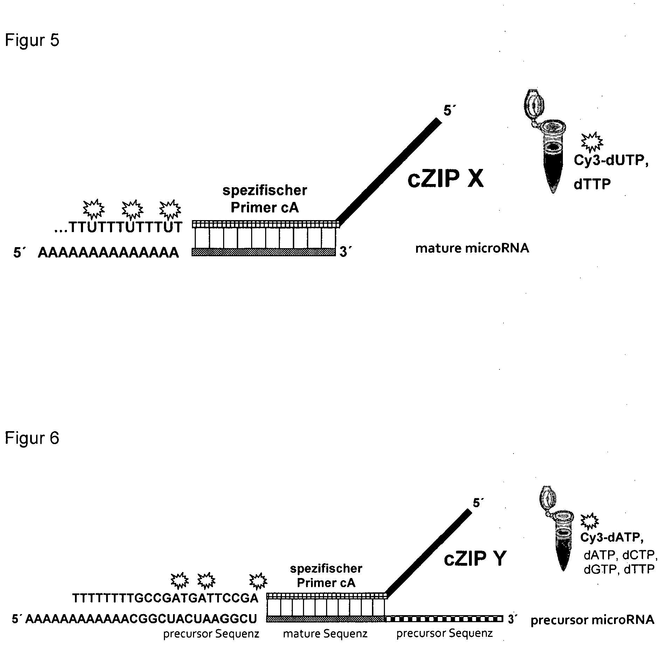

Figur 5 zeigt die Fluoreszenzmarkierung einer reifen mikroRNA mittels eines Primer-cZIP X-Polynucleotides gemäß Schritt h) unter Verwendung von Cy3-dUTP (Cy3-desoxy-Uridintriphosphat) und unmarkiertem dTTP (desoxy-Thymidintriphosphat).
Figur 6 zeigt die Fluoreszenzmarkierung einer Vorläufer-mikroRNA mittels eines Primer-cZIP Y-Polynucleotides gemäß Verfahrensschritt i) unter Verwendung von Cy3-dATP (Cy3-desoxy-Adenosintriphosphat) und unmarkierten dNTPs (desoxy-N ukleosidtriphosphate).
Figur 7 zeigt schematisch die Identifizierung von reifen und Vorläufer-mikroRNAs durch Bindung von markierten und strangverlängerten Primer-cZIP-Polynucleotiden an die an unterschiedlichen Positionen immobilisierten Gegen-ZIPs auf dem Nucleotid-Mikroarray-Träger.
Figur 8 zeigt ein Schema des Aufbaus von synthetischen mikroRNA-Templaten (T7-cDNA).
Figur 9 zeigt die spezifische Detektion von reifer mikroRNAs.
Figur 10 zeigt die spezifische Detektion von Vorläufer-mikroRNAs.
Figur 11 zeigt die spezifische Detektion ausgewählter reifer und Vorläufer-mikroRNAs.
Figur 12 zeigt den Vergleich der Signalintensitäten korrespondierender Gegen-ZIP/cZIP-Paare basierend auf der Fluoreszenzmarkierung von Vorläufer-Templaten. Korrespondierende Gegen-ZIP/cZIP-Paare der gleichen mikroRNA sind benachbart in gleichem Ton dargestellt.
Figur 13 zeigt den Vergleich der Signalintensitäten korrespondierender Gegen-ZIP/cZIP-Paare basierend auf der Fluoreszenzmarkierung von Vorläufer-Templaten. Korrespondierende Gegen-ZIP/cZIP-Paare der gleichen mikroRNA sind benachbart in gleichem Ton dargestellt.
Figur 14 zeigt den Vergleich der Fluoreszenzmarkierung von reifen Templaten (ZIP-X) und Vorläufer-Templaten (ZIP-Y) mit verschiedenen fluoreszenzmarkierten Desoxynukleotiden Cy3-dUTP bzw. Cy3-dATP. Korrespondierende Gegen-ZIP/cZIP-Paare pro mikroRNA sind benachbart in gleichem Ton dargestellt.
Figur 15 zeigt den Vergleich der Fluoreszenzmarkierung von Vorläufer-Templaten (ZIP-X) und reifen Templaten (ZIP-Y) mit verschiedenen fluoreszenzmarkierten Desoxynukleotiden Cy3-dATP beziehungsweise Cy3-dUTP. Korrespondierende Gegen-ZIP/cZIP-Paare der gleichen mikroRNA sind benachbart in gleichem Ton dargestellt.
Figur 16 zeigt die Detektion von reifen (variabel) und Vorläufer-(konstant) mikroRNA-Templat-Mengen über einen Bereich von vier Zehnerpotenzen.
Figur 17 zeigt die Detektion von reifen (konstant) und Vorläufer- (variabel) mikroRNA-Templat-Mengen über einen Bereich von vier Zehnerpotenzen.
Figur 18 zeigt die Detektion von reifen und Vorläufer-mikroRNAs in HeLa-Zellen.

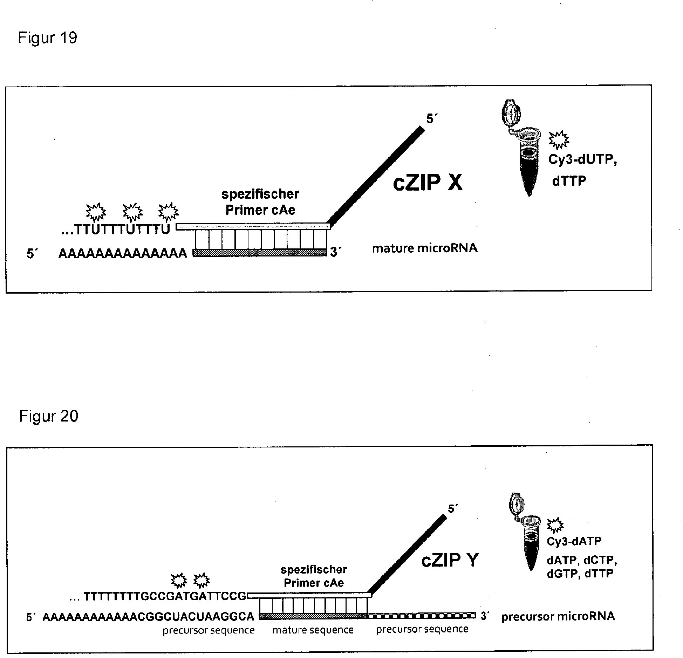

Figur 19 zeigt das Schema der Fluoreszenzmarkierung von einer reifen mikroRNA mittels eines erweiterten Primer-cZIP X-Polynucleotids, das eine Primernucleotidsequenz cAe aufweist. Die Fluoreszenzmarkierung der reifen mikroRNA erfolgt mit Cy3-dUTP (Cy3-desoxy-Uridintriphosphat) und unmarkiertem dTTP (desoxy-Thymidintriphosphat) unter Verwendung eines erweiterten Primer-cZIP X-Polynucleotids, das einen in 3'-Richtung um ein desoxy-Thymidintriphosphat verlängerten mikroRNA-spezifischen Primer cAe aufweist.
Figur 20 zeigt das Schema einer Fluoreszenzmarkierung von einer Vorläufer-mikroRNA mittels eines erweiterten Primer-cZIP Y-Polynucleotid, das eine Primernucleotidsequenz cAe aufweist. Die Fluoreszenzmarkierung der Vorläufer-mikroRNA erfolgt mit Cy3-dATP (Cy3-desoxy-Adenosintriphosphat) und unmarkierten dNTPs (desoxy-Nucleosidtriphosphate) unter Verwendung eines erweiterten Primer-cZIP Y-Polynucleotids, das einen in 3'-Richtung um ein desoxy-Thymidintriphosphat verlängerten mikroRNA-spezifischen Primer cAe aufweist.

Tabelle 1 zeigt eine Übersicht zum Versuchsaufbau und zu den Experimenten zur Etablierung der Methode mittels synthetischer mikroRNA-Templates.

Tabelle 2 zeigt ausgewählte mikroRNAs.

Tabelle 3 zeigt die Definition der mikroRNA-spezischen Primer mit cZIPs, d.h. die MSP-cZIP-Sets X und Y beziehungsweise die Primer cZIP X/Y-Polynukleotide.

Tabelle 4 zeigt eine spezielle Auswahl an synthetischen mikroRNA-Templaten.

### Beispiele:

### I. Versuchsaufbau:

In Tabelle 1 sind die Etablierungsexperimente zur Bestimmung der Selektivität und Spezifität synthetischer mikroRNA-Templates (Template: auch als Vorlage oder Matrizenstrang bezeichnet) zum Nachweis beziehungsweise der Unterscheidung von reifer (= mature) und Vorläufer (= precursor) mikroRNA aufgelistet. Ferner wurden Versuche mit mikroRNA aus Humanzellen durchgeführt.

Es werden folgende Abkürzungen verwendet:
Cy3-dUTP = Cy3-desoxy-Uridintriphosphat
Cy3-dATP = Cy3-desoxy-Adenosintriphosphat
dNTP = desoxy-Nukleosidtriphosphat
dCTP = desoxy-Cytidintriphosphat
dGTP = desoxy-Guanosintriphosphat
dTTP = desoxy-Thymidintriphosphat
M-MuLV = Moloney Mausleukämievirus
RNaseH = Ribonuklease H
IVT = In-vitro-Transcription
MSP = microRNA spezifischer Primer

**Tabelle 1**

| **Überprüfung von** | **Experiment** | **MSP-cZIP-Set X** | **MSP-cZIP-Set Y** |
|---|---|---|---|
| **1. Spezifität für mature bzw. precursor mikroRNA** | a) Fluoreszenzmarkierung von mature Templaten | Mature Template + Cy3-dUTP | Precursor Template + dNTPs (ohne Fluoreszenzmarkierung) |
| | b) Fluoreszenzmarkierung von precursor Templaten | Mature Template + dNTPs (ohne Fluoreszenzmarkierung) | Precursor Template + Cy3-dATP + dNTPs |
| | c) Fluoreszenzmarkierung von ausgewählten mature und precursor Templaten | Mature Template + Cy3-dUTP | Precursor Template + Cy3-dATP + dNTPs |
| **2. Vergleichbarkeit unterschiedlicher ZIP-Codes** | a) Fluoreszenzmarkierung von mature Templaten | Mature Template + Cy3-dUTP | Mature Template + Cy3-dUTP |
| | b) Fluoreszenzmarkierung von precursor Templaten | Precursor Template + Cy3-dATP + dNTPs | Precursor Template + Cy3-dATP + dNTPs |
| **3. Vergleichbarkeit unterschiedlicher Desoxynukleotide** | a) Fluoreszenzmarkierung von mature Templaten mit MSP-cZIP X, precursor Template mit MSP-cZIP Y | Mature Template + Cy3-dUTP | Precursor Template + Cy3-dATP + dNTPs |
| | b) Fluoreszenzmarkierung von precursor Templaten mit MSP-cZIP X, mature Template mit MSP-cZIP Y | Precursor Template + Cy3-dATP + dNTPs | Mature Template + Cy3-dUTP |
| **4. Sensitivität bzw. Detektionsbereich** | a) Fluoreszenzmarkierung von mature Templaten mit MSP-cZIP X, precursor Template mit MSP-cZIP Y | Mature Template: variierende Mengen (0,3 pmol / 0,03 pmol / 0,003 pmol) + Cy3-dUTP | Precursor Template: konstante Menge (3 pmol) + Cy3-dATP + dNTPs |
| | a) Fluoreszenzmarkierung von mature Templaten mit MSP-cZIP X, precursor Template mit MSP-cZIP Y | Mature Template: konstante Menge (3 pmol) + Cy3-dUTP | Precursor Template: variierende Mengen (0,3 pmol / 0,03 pmol / 0,003 pmol) + Cy3-dATP + dNTPs |
| **5. Übertragbarkeit auf mikroRNA aus Zellen** | | Mature mikroRNA + Cy3-dUTP | Precursor mikroRNA + Cy3-dATP + dNTPs |

### II. Versuchsdurchführung beziehungsweise Methodenbeschreibung:

Es wurden synthetische mikroRNA-Template für die folgenden zehn mikroRNAs (Tabelle 2) hergestellt, welche eine Rolle in der Tumorgenese spielen.

**Tabelle 2**

| **mature mikroRNA** | **precursor mikroRNA** |
|---|---|
| hsa-miR-16-5p | hsa-mir-16-1 |
| hsa-miR-21-5p | hsa-mir-21 |
| hsa-miR-92a-3p | hsa-mir-92a-1 |
| hsa-miR-100-5p | hsa-mir-100 |
| hsa-miR-19b-3p | hsa-mir-19b-1 |
| hsa-miR-206 | hsa-mir-206 |
| hsa-miR-139-5p | hsa-mir-139 |
| hsa-miR-29a-3p | hsa-mir-29a |
| hsa-miR-199b-5p | hsa-mir-199b |
| hsa-miR-99a-5p | hsa-mir-99a |

### a) Herstellung synthetischer mikroRNA-Templates

Für die ausgewählten mikroRNAs wurden jeweils synthetische mature bzw. precursor mikroRNA-Template hergestellt, welche analog zu einem Protokoll zur linearen Amplifizierung von mikroRNA aus Zellen [Mattie, M.D., et al., Optimized high-throughput mikroRNA expression profiling provides novel biomarker assessment of clinical prostate and breast cancer biopsies. Mol Cancer, 2006. 5: p. 24.] jeweils ein Poly A- 5'- Ende aufwiesen, d.h. Herstellung durch Umschreibung und Amplifizierung von T7-Promotor-gekoppelter komplementärer DNA mittels *in-vitro*-Transkription in polyA-mikroRNA.

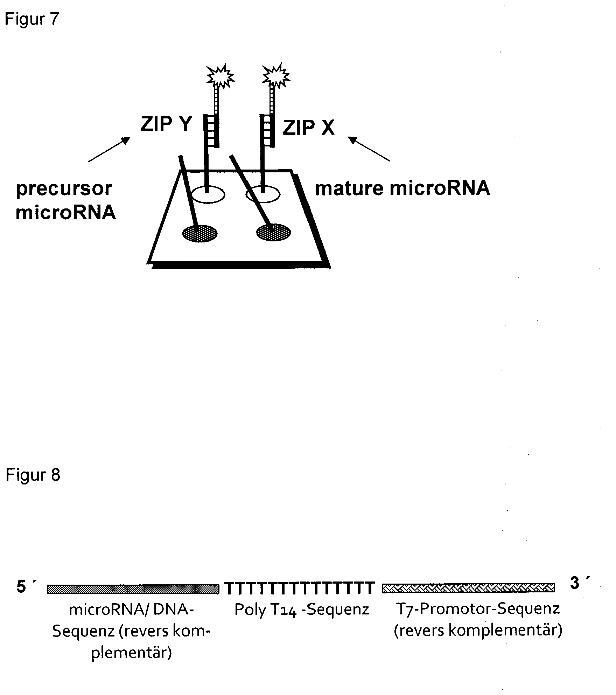

Zunächst wurde für die ausgewählten mikroRNAs jeweils die entsprechenden mature und precursor mikroRNA Nukleotidsequenzen aus der miRBase-Datenbank extrahiert und die hierzu revers komplementäre DNA-Sequenz am 3'-Ende mit einer poly-dT₁₄-Sequenz und der revers komplementären T7 RNA- Polymerase- Promotorsequenz verbunden (=T7-cDNA) (Figur 8).

Nach der Synthese der T7-cDNAs (Fa. Metabion) wurde zunächst der T7-Promotor vervollständigt. Hierzu wurden äquimolare Mengen an T7-Promoter-Oligonukleotid mit T7-cDNA vermischt, 3 min bei 95°C denaturiert und anschließend die beiden Stränge über 14 Stunden bei Raumtemperatur angelagert. Die Herstellung und Amplifikation der mikroRNAs erfolgte durch In-vitro-Transcription (IVT) der T7-cDNA. Hierzu wurde jeweils 1 µg der entsprechenden T7-cDNA mit 5 x T7 RNA-Polymerase-Transkriptionspuffer (Fermentas), 2 mM NTPs (GE Healthcare), 50 U RNaseOut (Invitrogen) und 30 U T7 RNA-Polymerase (Fermentas) versetzt. Die In-vitro-Transcription (IVT) erfolgte über 14 h bei 37°C, anschließend wurde die amplifizierte mikroRNA mit dem RNeasy Min Elute Cleanup Kit (Qiagen) gemäß des Protokolls des Herstellers aufgereinigt und photometrisch analysiert.

### b) Isolierung und Amplifizierung von mikroRNA aus Zellen:

Die Isolierung von mikroRNA aus der Zelllinie HeLa erfolgte mittels mirPremier mikroRNA Isolation Kit (Sigma-Aldrich), analog des Protokolls des Herstellers, ausgehend von tiefgefrorenen Pellets mit je 10 Millionen Zellen.

Die Amplifizierung von mikroRNA erfolgte mittels dem NCode miRNA Amplification System (Invitrogen) gemäß den Angaben des Herstellers, ausgehend von 30 ng angereichter mikroRNA, mit einer In-vitro-Transcription (IVT) - Reaktionsdauer von 16 h. Die amplifizierte mikroRNA wurde anschließend mittels RNeasy MinElute Cleanup Kit (Qiagen) aufgereinigt, gemäß des *RNA Cleanup and Concentration-* Protokolls des Herstellers.

### c) Herstellung mikroRNA-spezifischer Primer mit cZIP:

Für jede mikroRNA wurde ein mature mikroRNA-spezifischer Primer generiert, welcher am 5'-Ende in Kombination mit zwei unterschiedlichen cZIP-Code-Sequenzen, nämlich einem cZIP X- oder cZIP Y-Polynucleotid verbunden wurde (s. Figur 3 und MSP-cZIP-Set X bzw. Y, Tabelle 3). Es wurden dadurch mikroRNA-spezifische Primer mit cZIPs, d.h. MSP-cZIPs beziehungsweise Primer cZIP-Polynucleotide erhalten.

**Tabelle 3**

| **MSP-cZIP-Set X** | **MSP-cZIP-Set Y** |
|---|---|
| mi-16_ZIP C | mi-16_ZIP D |
| mi-21_ZIP E | mi-21_ZIP F |
| mi-92_ZIP G | mi-92_ZIP H |
| mi-100_ZIP I | mi-100_ZIP K |
| mi-19b_ZIP L | mi-19b_ZIP M |
| mi-206_ZIP N | mi-206_ZIP O |
| mi-139_ZIP P | mi-139_ZIP Q |
| mi-29a_ZIP R | mi-29a_ZIP S |
| mi-199b_ZIP T | mi-199b_ZIP U |
| mi-99a_ZIP W | mi-99a_ZIP X |

### d) cDNA-Synthese und spezifische Fluoreszenzmarkierung von mikroRNA:

cDNA-Synthese und Fluoreszenzmarkierung von mikroRNA erfolgte ausgehend von jeweils 50 ng jeder individuellen synthetischen mikroRNA bzw. 1.5 µg amplifizierter mikroRNA aus Zelllinien.

Zur mikroRNA wurden in einem Volumen von 10 µl je 0.25 pmol mikroRNA-spezifische cZIP-gekoppelte Oligonukleotide, nämlich MSP-cZIP beziehungesweise Primer cZIP-Polynukleotide, zugegeben, für 5 min bei 70°C denaturiert und die Lösung zur Anlagerung der MSP-cZIPs an die mikroRNA bei 50°C für 30 min inkubiert.

Die cDNA-Synthese und Fluoreszenzmarkierung erfolgte in einer Reversen Transkription durch Einbau verschiedener fluoreszenzmarkierter Desoxynukleotide Cy3- dATP und/oder Cy3- dUTP.

Der Einbau von Cy3-dUTP erfolgte in einem Gesamtvolumen von 20 µl, indem 5 x M-MuLV Reverse Transcriptase- Reaktionspuffer (Fermentas), 0.5 mM dTTP (Qiagen), 20 U RNaseOut (Invitrogen), 18.75 mM MgCl2 (Invitrogen), 40 U M-MuLV Reverse Transcriptase (Fermentas) und 0.05 mM Cy3-dUTP (GE Healthcare) zu dem angelagerten mikroRNA- Ansatz gegeben wurde.

Die Fluoreszenzmarkierung mit Cy3-dATP verlief analog, allerdings wurden anstatt 0,5 mM dTTP je 0,5 mM dCTP, dGTP, dTTP und 0,08 mM dATP (Qiagen) verwendet, weiterhin 0,05 mM Cy3-dATP (Perkin Elmer) statt 0,05 mM Cy3-dUTP.

Die Durchführung der Reversen Transkription erfolgte für 1.5 h bei 37 °C. Anschließend wurde das Enzym bei 70°C für 10 min hitzeinaktiviert. Nach Zugabe von 2 U RNase H (Invitrogen) zu jeder Probe zum Abbau der RNA für 20 min bei 37°C wurden die Proben vereinigt und die cDNA mittels QIAquick Nucleotide Removal Kit (Qiagen) nach dem *Nucleotide Removal Protocol* aufgereinigt.

### e) Herstellung von ZIP-Code-DNA-Mikroarrays:

Zur Herstellung von DNA-Mikroarrays, auf welchen die ZIP-Codes/Gegen-ZIPs an definierten Positionen immobilisiert sind, wurden D-DNA-basierte ZIP-Code-Oligonukleotide mit 3'-Amino-C7-Modifikation in 1x Spottingpuffer (Nexterion Spot I/ Spot III, Schott) auf eine Konzentration von 50 µM eingestellt und mittels Mikroarray-Spotter (MikroGrid II, Digilab) auf Epoxy- beschichteten Glasobjektträgern *(Slides)* (Nexterion Slide E, Schott) gedruckt. Nach dem Spottingprozess wurden die gedruckten Oligonukleotide entsprechend den Angaben des Herstellers auf den Slides immobilisiert. Die Slides wurden unmittelbar vor ihrer Verwendung entsprechend den Angaben des Herstellers geblockt.

### f) Hybridisierung, Scannen und Datenanalyse:

Die Hybridisierung der Probenlösung auf die ZIP-Code-Arrays erfolgte in 4 x SSC (Natriumchlorid-Natriumcitrat-Lösung) und 0.1 % SDS (Natriumdodecylsulfat) in einem Volumen von 45 µl unter Verwendung von LifterSlips (Erie Scientific) über Nacht bei 65 °C in einer Hybridisierungskammer.

Im Anschluss an die Hybridisierung wurden die Slides entsprechend den Angaben des Herstellers gewaschen und zur Detektion der Fluoreszenzfarbstoffe der DNA-Mikroarray mit einem Axon GenePix 4300A-Scanner (Molecular Devices) eingescannt. Zur Spotquantifizierung wurde die GenePixPro 7-Software (Molecular Devices) herangezogen, die weitere Datenanalyse basierte auf den gemittelten Hintergrund-korrigierten Median-Werten.

### III. Versuchsergebnisse:

### Ad 1. Überprüfung der Spezifität für mature bzw. precursor mikroRNA

Ermittlung der Spezifität der Methode bezüglich des eindeutigen Nachweises bzw. Unterscheidung von mature bzw. precursor mikroRNA, zum einen durch ausschließliche Fluoreszenzmarkierung von synthetischen mature bzw. precursor mikroRNA-Templaten, des weiteren durch definierte Kombination bestimmter mature und precursor Template und deren Detektion.

### 1a) Detektion von mature mikroRNA

Fluoreszenzmarkierung von synthetischen mature Templaten mit Cy3-dUTP (und dTTP) unter Verwendung des MSP-cZIP Sets X bzw. von synthetischen precursor Templaten mit ausschließlich unmarkierten Desoxynukleotiden und des MSP-cZIP- Sets Y in getrennten Ansätzen, anschließend gleichzeitige Hybridisierung auf dem ZIP-Code-Array.

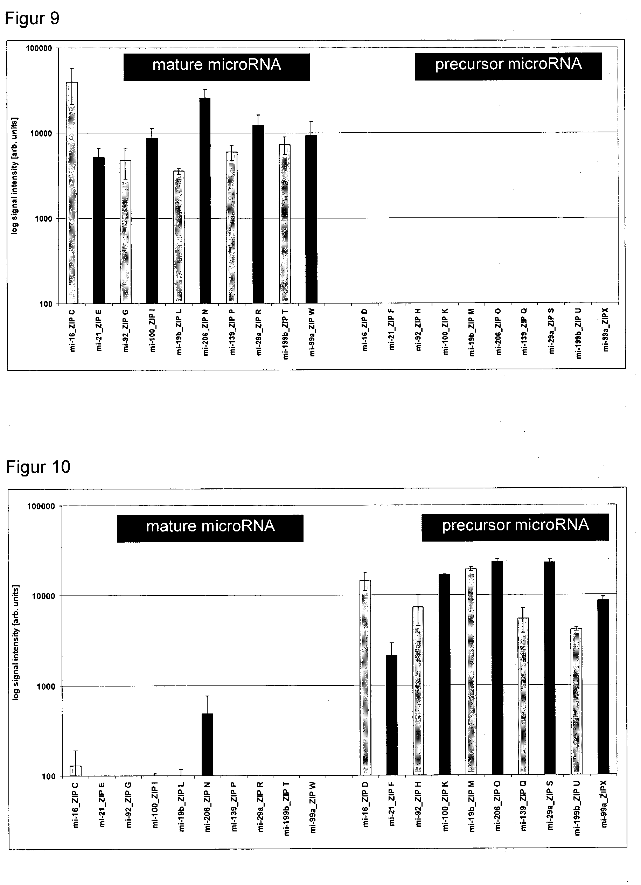

In Figur 9 sind die logarithmierten Signalintensitäten dieser Hybridisierung dargestellt. Das Ergebnis zeigt die eindeutige Spezifität zur Detektion von mature mikroRNA in 100 % aller Fälle. Für precursor ZIPs wurden keine Signale detektiert.

### 1b) Detektion von precursor mikroRNA

Fluoreszenzmarkierung von synthetischen precursor Templaten mit Cy3-dATP und dNTPs unter Verwendung des MSP-cZIP Sets Y bzw. synthetischen mature Templaten mit ausschließlich unmarkierten Desoxynukleotiden unter Verwendung des MSP-cZIP Sets X in getrennten Ansätzen, anschließend parallele Hybridisierung auf den ZIP-Code-Array.

In Figur 10 sind die logarithmisch aufgetragenen Signalintensitäten aus diesem Experiment dargestellt. Das Ergebnis der Hybridisierung weist keine signifikanten Signale für mature mikroRNA auf und bestätigt somit die Spezifität der Methode zur ausschließlichen Detektion von precursor mikroRNA.

### 1c) Detektion bestimmter mature und precursor mikroRNAs

Kombination einer definierten Auswahl verschiedener synthetischer mature und precursor mikroRNA- Template (Tabelle 4) ; Fluoreszenzmarkierung von synthetischen mature Templaten mit Cy3-dUTP (und dTTP) unter Verwendung des MSP-cZIP Sets X bzw. von synthetischen precursor Templaten mit Cy3-dATP und dNTPs unter Verwendung des MSP-cZIP Sets Y in getrennten Ansätzen, anschließend parallele Hybridisierung auf den ZIP-Code-Array.

**Tabelle 4**

| **Vorhandene mikroRNAs:** | **Fehlende mikroRNAs:** |
|---|---|
| miR-16_ZIP C, mir-16_ZIP D, miR-21_ZIP E, mir-92_ZIP H, miR-100_ZIP I, mir-19b_ZIP M, miR-206_ZIP N, mir-29a_ZIP S, miR-199b_ZIP T, miR-99a_ZIP W | mir-21_ZIP F, miR-92_ZIP G, mir-100_ZIP K, miR-19b_ZIP L, mir-206_ZIP O, miR-139_ZIP P, mir-139_ZIP Q, miR-29a_ZIP R, mir-199b_ZIP U, mir-99a_ZIP X |

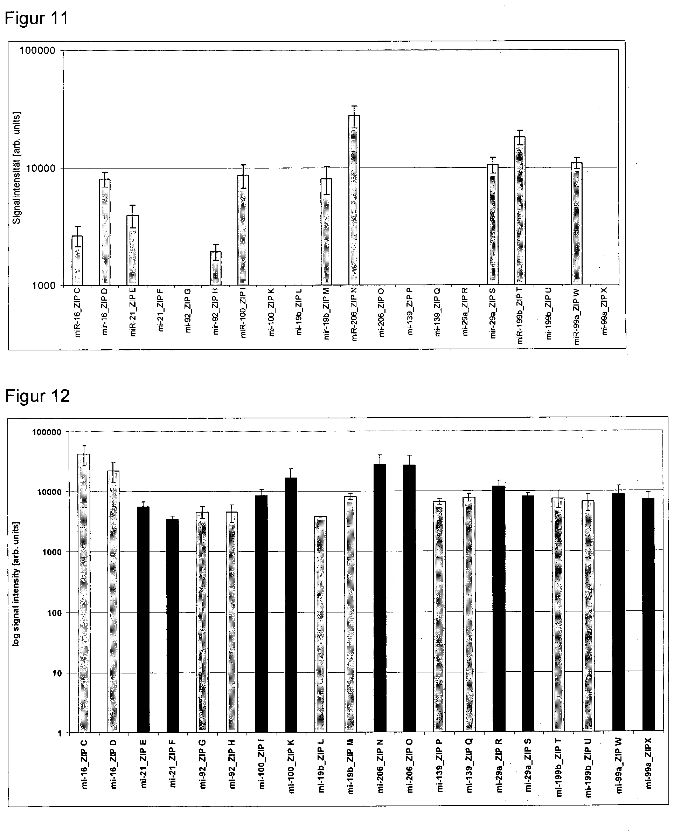

Figur 11 zeigt die logarithmisch aufgetragenen Signalintensitäten dieser Hybridisierung. Das Ergebnis verdeutlicht die Spezifität zur Detektion von mature und precursor mikroRNA durch direkten Vergleich der verwendeten synthetischen mikroRNA-Template (Tabelle 4) mit den beobachteten Signalen.

### Ad 2. Überprüfung der Vergleichbarkeit unterschiedlicher ZIP-Codes

Ermittlung der Vergleichbarkeit unterschiedlicher ZIP-Code-Sequenzen durch Fluoreszenzmarkierung identischer synthetischer mikroRNA-Template unter Verwendung identischer mikroRNA-spezifischer Primer, jedoch in Kombination mit zwei unterschiedlichen cZIP-Codes.

### 2a) Fluoreszenzmarkierung von mature mikroRNA

Fluoreszenzmarkierung von synthetischen mature Templaten mit Cy3-dUTP (und dTTP) unter Verwendung des MSP-cZIP Sets X, sowie des MSP-cZIP Sets Y in getrennten Ansätzen, anschließend gleichzeitige Hybridisierung auf dem ZIP-Code-Array.

In Figur 12 sind die logarithmisch aufgetragenen Signalintensitäten korrespondierender ZIP-Codes dargestellt.

Beim Vergleich der korrespondierenden ZIP-Paare für die jeweilige mikroRNA wurden im Idealfall identische Signalintensitäten erwartet; unter Berücksichtigung der Standardabweichung aus der Hybridisierung von Triplikaten, konnten bei 9 von 10 ZIP-Paaren der überprüften mikroRNAs vergleichbare Signalintensitäten beobachtet werden.

### 2b) Fluoreszenzmarkierung von precursor mikroRNA

Fluoreszenzmarkierung von synthetischen precursor Templaten mit Cy3-dATP und dNTPs unter Verwendung des MSP-cZIP Sets X sowie des MSP-cZIP Sets Y in getrennten Ansätzen, anschließend parallele Hybridisierung auf dem ZIP-Code-Array.

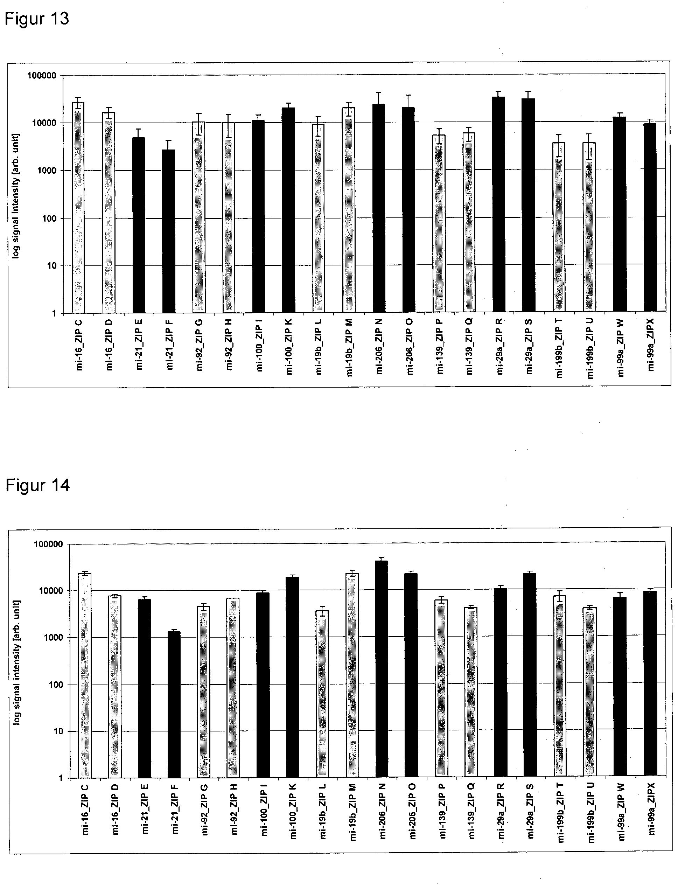

Figur 13 zeigt das Ergebnis des Vergleichs der logarithmisch aufgetragenen Signalintensitäten von korrespondierenden ZIP-Paaren für die jeweilige mikroRNA. In diesem Experiment konnten vergleichbare Signalintensitäten bei 9 von 10 ZIP-Paaren beobachtet werden.

### Ad 3. Überprüfung der Vergleichbarkeit unterschiedlicher fluoreszenz-markierter Desoxynukleotide

Überprüfung der Vergleichbarkeit der Verwendung zweier unterschiedlicher fluoreszenzmarkierter Desoxynukleotide, d.h. Cy3-dUTP bzw. Cy3-dATP, zur Fluoreszenzmarkierung von mature bzw. precursor mikroRNA durch Vergleich der Signalintensitäten von mature und precursor mikroRNA nach deren Fluoreszenzmarkierung unter Verwendung korrespondierender MSP-cZIPs.

### 3a) Vergleich mature (ZIP-Set X) vs. precursor mikroRNA (ZIP-Set Y)

Fluoreszenzmarkierung von synthetischen mature Templaten mit Cy3-dUTP (und dTTP) unter Verwendung des MSP-cZIP Sets X bzw. von synthetischen precursor Templaten mit Cy3-dATP und dNTPs unter Verwendung des MSP-cZIP Sets Y in getrennten Ansätzen, anschließend parallele Hybridisierung auf den ZIP-Code-Array.

Das Ergebnis der Hybridisierung in Figur 14 zeigt vergleichbare Signalintensitäten für korrespondierende mature und precursor mikroRNAs in der Mehrzahl aller Fälle (7/10).

### 3b) Vergleich precursor (ZIP-Set X) vs. mature mikroRNA (ZIP-Set Y)

Fluoreszenzmarkierung von synthetischen precursor Templaten mit Cy3-dATP und dNTPs unter Verwendung des MSP-cZIP Sets X bzw. von synthetischen mature Templaten mit Cy3-dUTP (und dTTP) unter Verwendung des MSP-cZIP Sets Y in getrennten Ansätzen, anschließend parallele Hybridisierung auf den ZIP-Code-Array.

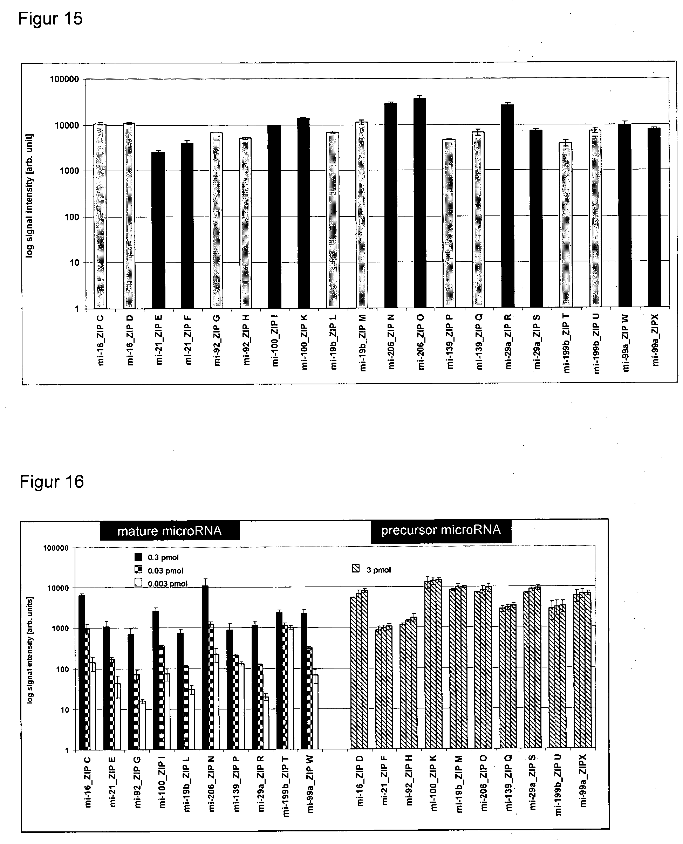

Wie in Figur 15 ersichtlich, konnten vergleichbare Signalintensitäten in 9 von 10 Fällen von korrespondierenden mature und precursor mikroRNAs bestätigt werden.

### Ad 4. Überprüfung der Sensitivität bzw. des Detektionsbereiches

Zur Überprüfung der quantitativen Eigenschaften des Systems bzw. der Sensitivität der mikroRNA-Detektionsmethode wurde die Größenordnung des Detektionsbereiches durch Vergleich der Signalintensitäten verschiedener Template-Einsatzmengen über einen Bereich von vier Zehnerpotenzen ermittelt. Fluoreszenzmarkierung von synthetischen mature Templaten mit Cy3-dUTP (und dTTP) unter Verwendung des MSP-cZIP-Sets X bzw. synthetische precursor Template mit Cy3-dATP und dNTPs unter Verwendung des MSP-cZIP-Sets Y.

### 4a) mature Template variabel bzw. precursor Template konstant

Fluoreszenzmarkierung von drei unterschiedlichen Mengen an synthetischen mature Templaten (Zehnfachverdünnungen über einen Bereich von 0.3 bis 0.003 pmol) bzw. von konstanter synthetischer precursor Template-Menge (3 pmol).

Die Ergebnisse in Figur 16 zeigen für mature Template logarithmisch abnehmende Signalintensitäten (außer bei mi-199b) und konstante Signalintensitäten bei precursor Templaten.

Das System wies in diesem Experiment somit einen Detektionsbereich von über mindestens drei Zehnerpotenzen für 9 von 10 der untersuchten mikroRNAs auf (bzw. vier Zehnerpotenzen für 6 von 10).

### 4b) mature Template konstant bzw. precursor Template variabel

Fluoreszenzmarkierung einer konstanten Menge (3 pmol) an synthetischen mature Templaten bzw. von drei variablen Mengen (Zehnfachverdünnungen über einen Bereich von 0.3 bis 0.003 pmol) an synthetischen precursor Templaten.

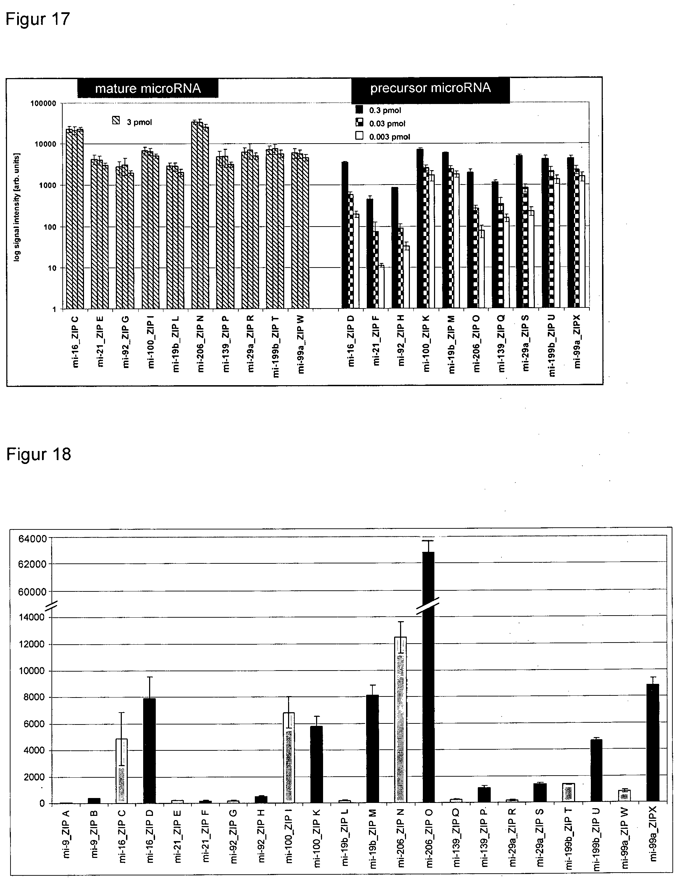

Das Ergebnis in Figur 17 zeigt konstante Signalintensitäten bei mature mikroRNA und logarithmisch abnehmende Signale bei den precursor Templaten.

Somit konnte ein Detektionsbereich von über mindestens drei Zehnerpotenzen in 7 von 10 Fällen der untersuchten mikroRNAs konstatiert werden (bzw. vier Zehnerpotenzen für 5 von 10 Fällen).

### Ad 5. Überprüfung der Anwendung zur Detektion von mikroRNA in humanen Zellen

Im Anschluss an die Etablierung der Methode zur zeitgleichen Detektion bzw. Unterscheidung von mature und precursor mikroRNA auf der Basis synthetischer mikroRNA-Template wurde deren Übertragbarkeit zur Detektion von mikroRNA in humanen Zellen anhand der Tumor- Zelllinie HeLa überprüft.
Hierzu wurde amplifizierte mikroRNA aus der HeLa-Zelllinie fluoreszenzmarkiert, d.h. darin enthaltene mature mikroRNA mit Cy3-dUTP (und dTTP) unter Verwendung des MSP-cZIP-Sets X bzw. precursor mikroRNA mit Cy3-dATP und dNTPs mit MSP-cZIP-Set Y.

Die Signalintensitäten der mikroRNA-Detektion in HeLa-Zellen sind in Figur 18 dargestellt; es konnten individuelle mature und precursor mikroRNAs detektiert werden.

Zur Validierung der erhaltenen Ergebnisse der mikroRNA-Detektion in HeLa-Zellen mittels ZIP-Code-Array wurde die quantitative Real-Time PCR als unabhängige Methode herangezogen. Die Präsenz bzw. Absenz/Expression von jeweils mature und precursor Form von drei der zehn mikroRNAs (mi-16, mi-199b, mi-99a) in HeLa-Zellen wurde mittels qRT-PCR überprüft, mit den DNA-Mikroarray-Ergebnissen korreliert und diese somit positiv bestätigt.

## Patentansprüche

1. Verfahren zum differenzierten Nachweis von in einer Probenlösung vorhandenen, mindestens zwei unterschiedlichen ersten und zweiten RNA-Molekülen, die jeweils eine identische Polynucleotidsequenz A aufweisen, wobei das Verfahren folgende Schritte umfasst:
a) Bereitstellen einer Probenlösung, die die mindestens zwei unterschiedlichen ersten und zweiten RNA-Moleküle aufweist, wobei das erste RNA-Molekül die Polynucleotidsequenz A ist und das zweite RNA-Molekül die Polynucleotidsequenz A und unmittelbar anschließend 5'-wärts ein Nucleotidelement P aufweist,
b) Bereitstellen eines eine Primernukleotidsequenz cA und eine cZIP X-Nukleotidsequenz aufweisenden Primer-cZIP X-Polynucleotids, wobei die Primernukleotidsequenz cA komplementär zu einem endständigen 5'-Abschnitt der Polynucleotidsequenz A ist, und eines die Primernukleotidsequenz cA und eine cZIP Y-Nukleotidsequenz aufweisenden Primer-cZIP Y-Polynucleotids,
c) Bereitstellen eines Nucleotid-Microarrays umfassend mindestens zwei unterschiedliche erste und zweite auf einem Nucleotid-Microarrayträger an unterschiedlichen Positionen immobilisierte Gegen-ZIPs, wobei das erste Gegen-ZIP komplementär zu der cZIP X-Nucleotidsequenz und das zweite Gegen-ZIP komplementär zu der cZIP Y-Nucleotidsequenz ist,
d) Adenylierung der in der Lösung aus Schritt a) vorhandenen ersten und zweiten RNA-Moleküle, wobei eine erste und zweite adenylierte RNA-Moleküle aufweisende Lösung erhalten wird,
e) Teilen der die ersten und zweiten adenylierten RNA-Moleküle aufweisenden Lösung aus Schritt d) in eine erste Lösung und eine zweite Lösung,
f) Versetzen der ersten Lösung mit dem in Schritt b) bereitgestellten Primer-cZIP X-Polynucleotid und Durchführung einer Hybridisierung mit dem ersten und zweiten adenylierten RNA-Molekül, wobei eine Lösung A1 erhalten wird,
g) Versetzen der zweiten Lösung mit dem in Schritt b) bereitgestellten Primer cZIP Y-Polynucleotid und Durchführung einer Hybridisierung mit dem ersten und zweiten adenylierten RNA-Molekül, wobei eine Lösung B1 erhalten wird,
h) Versetzen der in Schritt f) erhaltenen Lösung A1 mit mindestens einem ersten Nucleosidtriphosphat, das mit einem Fluoreszenzfarbstoff markiert ist, wobei das erste Nucleosidtriphosphat ausgewählt ist aus desoxy-Thymidintriphosphat und desoxy-Uridintriphosphat und Durchführung einer enzymatischen Strangverlängerung des hybridisierten Primer-cZIP X-Polynukleotids, wobei eine Lösung A2 erhalten wird,
i) Versetzen der in Schritt g) erhaltenen Lösung B1 mit mindestens einem zweiten Nucleosidtriphosphat, das mit einem Fluoreszenzfarbstoff markiert ist, und wobei das zweite Nucleosidtriphosphat ausgewählt ist aus der Gruppe bestehend aus desoxy-Guanosintriphosphat, desoxy-Adenosintriphosphat und desoxy-Cytidintriphosphat, und Durchführung einer enzymatischen Strangverlängerung des hybridisierten Primer-cZIP Y-Polynucleotids, wobei eine Lösung B2 erhalten wird,
j) Inkontaktbringen der in den Schritten h) und i) erhaltenen Lösungen A2 und B2 mit dem in Schritt c) bereitgestellten Nucleotid-Mikroarray und Durchführung einer Hybridisierung der Primer-cZIP X- und Primer-cZIP Y-Polynucleotide mit den ersten und zweiten Gegen-ZIPs und
k) differenziertes Nachweisen der mindestens zwei unterschiedlichen ersten und zweiten RNA-Moleküle durch eine Fluoreszenzdetektion.

2. Verfahren nach Anspruch 1 , wobei die in den Schritten h) und i) erhaltenen Lösungen A2 und B2 vor Schritt j) vereinigt werden, so dass eine vereinigte Lösung C erhalten wird.

3. Verfahren nach einem der Ansprüche 1 bis 2, wobei die mindestens zwei unterschiedlichen ersten und zweiten RNA-Moleküle mikroRNA-Moleküle sind.

4. Verfahren nach einem der vorhergehenden Ansprüche, wobei das erste RNA-Molekül eine reife mikroRNA und das zweite RNA-Molekül eine Vorläufer-mikroRNA ist.

5. Verfahren nach einem der vorhergehenden Ansprüche, wobei das Primer-cZIP X-Polynucleotid und das Primer-cZIP Y-Polynucleotid eine DNA ist.

6. Verfahren nach einem der vorhergehenden Ansprüche, wobei zur Bereitstellung der Probenlösung gemäß Schritt a) eine Amplifikation des ersten und zweiten RNA-Moleküls erfolgt.

7. Verfahren nach einem der vorhergehenden Ansprüche, wobei während oder nach der Adenylierung gemäß Schritt d) eine Amplifikation der mindestens zwei unterschiedlichen ersten und zweiten adenylierten RNA-Moleküle erfolgt.

8. Verfahren nach einem der vorhergehenden Ansprüche, wobei nach Durchführung der Hybridisierung gemäß der Schritte f) und g) sowie der Strangverlängerung gemäß der Schritte h) und i) ein Abbau der ersten und zweiten RNA-Moleküle durch eine Ribonuklease durchgeführt wird.

9. Verfahren nach einem der vorhergehenden Ansprüche, wobei das erste und das zweite Nucleosidtriphosphat mit demselben Fluoreszenzfarbstoff markiert sind.

10. Verfahren nach einem der vorhergehenden Ansprüche, wobei der Fluoreszenzfarbstoff ein Cyaninfarbstoff, insbesondere Cy3, ist.

11. Kit zur Durchführung eines Verfahrens nach einem der Ansprüche 1 bis 10 zum differenzierten Nachweis von in einer Probenlösung vorhandenen, mindestens zwei unterschiedlichen ersten und zweiten RNA-Molekülen, die jeweils eine identische Polynucleotidse-quenz A aufweisen, enthaltend:
a1) einen Nucleotid-Microarray umfassend mindestens zwei unterschiedliche erste und zweite auf dem Nucleotid-Microarrayträger an unterschiedlichen Positionen immobilisierte Gegen-ZIPs,
b1) ein eine Primernukleotidsequenz cA und eine cZIP X-Nukleotidsequenz aufweisendes Primer-cZIP X-Polynucleotid, wobei die Primernukleotidsequenz cA komplementär zu einem endständigen 5'-Abschnitt der Polynucleotidsequenz A ist und wobei die cZIP X-Nukleotidsequenz komplementär zu dem ersten Gegen-ZIP des Nucleotid-Mikroarrays ist, und
c1) ein die Primernukleotidsequenz cA und eine cZIP Y-Nukleotidsequenz aufweisendes Primer-cZIP Y-Polynucleotid und wobei die cZIP Y-Nukleotidsequenz komplementär zu dem zweiten Gegen-ZIP des Nucleotid-Mikroarrays ist und
enthaltend eine Lösung I, die mindestens ein mit einem Fluoreszenzfarbstoff markiertes erstes Nucleosidtriphosphat und eine Lösung II, die mindestens ein mit einem Fluoreszenzfarbstoff markiertes zweites Nucleosidtriphosphat enthält, wobei das erste Nucleosidtriphosphat ausgewählt ist aus der Gruppe bestehend aus desoxy-Uridintriphosphat und desoxy-Thymidintriphosphat und wobei das zweite Nucleosidtriphosphat ausgewählt ist aus der Gruppe bestehend aus desoxy-Guanosintriphosphat, desoxy-Adenosintriphosphat und desoxy-Cytidintriphosphat.

12. Kit nach Anspruch 11, wobei der Kit mindestens ein Enzym für eine Polyadenylierung und mindestens ein Enzym für die Strangverlängerung enthält.

## Claims

1. Method for differentiated detection of at least two different first and second RNA molecules that are present in a sample solution and each comprise an identical polynucleotide sequence A, wherein the method comprises the following steps:
a) providing a sample solution that comprises the at least two different first and second RNA molecules, wherein the first RNA molecule is the polynucleotide sequence A and the second RNA molecule comprises the polynucleotide sequence A and, directly adjacent on the 5' side, a nucleotide element P;
b) providing a primer cZIP X-polynucleotide, which comprises a primer nucleotide sequence cA and a cZIP X-nucleotide sequence, wherein the primer nucleotide sequence cA is complementary to a 5'-terminal section of polynucleotide sequence A, and a primer-cZIP Y-polynucleotide that comprises the primer nucleotide sequence cA and a cZIP Y-nucleotide sequence;
c) providing a nucleotide micro-array comprising at least two different first and second counter-ZIPs that are immobilised on a nucleotide micro-array carrier at different positions, wherein the first counter-ZIP is complementary to the cZIP X-nucleotide sequence and the second counter-ZIP is complementary to the cZIP Y-nucleotide sequence;
d) adenylation of the first and second RNA molecules that are present in the solution from step a), whereby a solution comprising first and second adenylated RNA molecules is obtained;
e) splitting the solution comprising the first and second adenylated RNA molecules from step d) into a first solution and a second solution;
f) adding, to the first solution, the primer-cZIP X-polynucleotide provided in step b) and performing a hybridisation with the first and second adenylated RNA molecule, whereby a solution A1 is obtained;
g) adding, to the second solution, the primer cZIP Y-polynucleotide provided in step b) and performing a hybridisation with the first and second adenylated RNA molecule, whereby a solution B1 is obtained;
h) adding, to the solution A1 obtained in step f), at least one first nucleoside triphosphate labelled with a fluorescence dye, wherein the first nucleoside triphosphate is selected from deoxy-thymidine triphosphate and deoxy-uridine triphosphate, and performing an enzymatic strand elongation of the hybridised primer-cZIP X-polynucleotide, whereby a solution A2 is obtained;
i) adding, to the solution B1 obtained in step g), at least one second nucleoside triphosphate labelled with a fluorescence dye, wherein the second nucleoside triphosphate is selected from the group consisting of deoxy-guanosine triphosphate, deoxy-adenosine triphosphate, and deoxy-cytidine triphosphate, and performing an enzymatic strand elongation of the hybridised primer-cZIP Y-polynucleotide, whereby a solution B2 is obtained;
j) contacting the solutions A2 and B2 obtained in steps h) and i) with the nucleotide micro-array provided in step c), and performing a hybridisation of the primer-cZIP X- and primer-cZIP Y-polynucleotides with the first and second counter-ZIPs; and
k) differentiated detection of the at least two different first and second RNA molecules through a fluorescence detection.

2. Method according to claim 1, wherein the solutions A2 and B2 obtained in steps h) and i), respectively, are being combined before step j) so as to obtain a combined solution C.

3. Method according to any one of the claims 1 to 2, wherein the at least two different first and second RNA molecules are microRNA molecules.

4. Method according to any one of the preceding claims, wherein the first RNA molecule is a mature microRNA and the second RNA molecule is a precursor microRNA.

5. Method according to any one of the preceding claims, wherein the primer-cZIP X-polynucleotide and the primer-cZIP Y-polynucleotide is a DNA.

6. Method according to any one of the preceding claims, wherein an amplification of the first and second RNA molecule takes place in order to provide the sample solution according to step a).

7. Method according to any one of the preceding claims, wherein an amplification of the at least two different first and second adenylated RNA molecules takes place during or after the adenylation according to step d).

8. Method according to any one of the preceding claims, wherein a degradation of the first and second RNA molecules by a ribonuclease is performed after performing the hybridisation according to steps f) and g) and the strand elongation according to steps h) and i).

9. Method according to any one of the preceding claims, wherein the first and the second nucleoside triphosphate are labelled with the same fluorescence dye.

10. Method according to any one of the preceding claims, wherein the fluorescence dye is a cyanine dye, in particular Cy3.

11. Kit for performing a method according to any one of the claims 1 to 10 for differentiated detection of at least two different first and second RNA molecules that are present in a sample solution and each comprise an identical polynucleotide sequence A, containing:
a1) a nucleotide micro-array comprising at least two different first and second counter-ZIPs that are immobilised on the nucleotide micro-array carrier at different positions;
b1) a primer-cZIP X-polynucleotide that comprises a primer nucleotide sequence cA and a cZIP X-nucleotide sequence, wherein the primer nucleotide sequence cA is complementary to a 5'-terminal section of polynucleotide sequence A and wherein the cZIP X-nucleotide sequence is complementary to the first counter-ZIP of the nucleotide micro-array; and
c1) a primer-cZIP Y-polynucleotide comprising the primer nucleotide sequence cA and a cZIP Y-nucleotide sequence, and wherein the cZIP Y-nucleotide sequence is complementary to the second counter-ZIP of the nucleotide micro-array; and
containing a solution I, which contains at least one first nucleoside triphosphate that is labelled with a fluorescence dye, and a solution II, which contains at least one second nucleoside triphosphate that is labelled with a fluorescence dye, wherein the first nucleoside triphosphate is selected from the group consisting of deoxy-uridine triphosphate and deoxy-thymidine triphosphate, and wherein the second nucleoside triphosphate is selected from the group consisting of deoxy-guanosine triphosphate, deoxy-adenosine triphosphate, and deoxy-cytidine triphosphate.

12. Kit according to claim 11, wherein the kit contains at least one enzyme for a polyadenylation and at least one enzyme for the strand elongation.

## Revendications

1. Procédé de dépistage différencié d'au moins deux première et seconde molécules d'ARN différentes, présentes dans une solution d'échantillon, qui présentent chacune une séquence polynucléotidique identique A, dans lequel le procédé comprend les étapes suivantes :
a) mise à disposition d'une solution d'échantillon qui présente les au moins deux première et seconde molécules d'ARN différentes, dans lequel la première molécule d'ARN est la séquence polynucléotidique A et la seconde molécule d'ARN présente la séquence polynucléotidique A et directement à la suite vers 5' un élément nucléotidique P,
b) mise à disposition d'un polynucléotide amorce-cZIP X présentant une séquence nucléotidique d'amorce cA et une séquence nucléotidique cZIP X, dans lequel la séquence nucléotidique d'amorce cA est complémentaire à une section en 5' terminale de la séquence polynucléotidique A, et d'un polynucléotide amorce-cZIP Y présentant la séquence nucléotidique d'amorce cA et une séquence nucléotidique cZIP Y,
c) mise à disposition d'un microréseau nucléotidique comprenant au moins deux premier et second contre-ZIP différents, immobilisés à différentes positions sur un support de microréseau nucléotidique, dans lequel le premier contre-ZIP est complémentaire à la séquence nucléotidique cZIP X et le second contre-ZIP est complémentaire à la séquence nucléotidique cZIP Y,
d) adénylation des première et seconde molécules d'ARN présentes dans la solution de l'étape a), dans lequel une solution présentant des première et seconde molécules d'ARN adénylées est obtenue,
e) division de la solution présentant les première et seconde molécules d'ARN adénylées de l'étape d) en une première solution et une seconde solution,
f) mélange de la première solution avec le polynucléotide amorce-cZIP X mis à disposition à l'étape b) et réalisation d'une hybridation avec les première et seconde molécules d'ARN adénylées, dans lequel une solution A1 est obtenue,
g) mélange de la seconde solution avec le polynucléotide amorce-cZIP Y mis à disposition à l'étape b) et réalisation d'une hybridation avec les première et seconde molécules d'ARN adénylées, dans lequel une solution B1 est obtenue,
h) mélange de la solution A1 obtenue à l'étape f) avec au moins un premier nucléoside triphosphate qui est marqué avec un colorant de fluorescence, dans lequel le premier nucléoside triphosphate est sélectionné parmi le désoxy-thymidine triphosphate et le désoxy-uridine triphosphate, et réalisation d'un prolongement de brin enzymatique du polynucléotide amorce-cZIP X hybridé, dans lequel une solution A2 est obtenue,
i) mélange de la solution B1 obtenue à l'étape g) avec au moins un second nucléoside triphosphate qui est marqué avec un colorant de fluorescence, et dans lequel le second nucléoside triphosphate est sélectionné parmi le groupe constitué du désoxy-guanosine triphosphate, désoxy-adénosine triphosphate et désoxy-cytidine triphosphate, et réalisation d'un prolongement de brin enzymatique du polynucléotide amorce-cZIP Y hybridé, dans lequel une solution B2 est obtenue,
j) mise en contact des solutions A2 et B2 obtenues aux étapes h) et i) avec le microréseau nucléotidique mis à disposition à l'étape c) et réalisation d'une hybridation des polynucléotides amorce-cZIP X et amorce-cZIP Y avec les premier et second contre-ZIP et
k) dépistage différencié des au moins deux première et seconde molécules d'ARN différentes par une détection de fluorescence.

2. Procédé selon la revendication 1, dans lequel les solutions A2 et B2 obtenues aux étapes h) et i) sont réunies avant l'étape j) de sorte qu'une solution C réunie est obtenue.

3. Procédé selon une des revendications 1 à 2, dans lequel les au moins deux première et seconde molécules d'ARN différentes sont des molécules de microARN.

4. Procédé selon une des revendications précédentes, dans lequel la première molécule d'ARN est un microARN mûr et la seconde molécule d'ARN est un microARN précurseur.

5. Procédé selon une des revendications précédentes, dans lequel le polynucléotide amorce-cZIP X et le polynucléotide amorce-cZIP Y sont un ADN.

6. Procédé selon une des revendications précédentes, dans lequel une amplification des première et seconde molécules d'ARN a lieu pour la mise à disposition de la solution d'échantillon selon l'étape a).

7. Procédé selon une des revendications précédentes, dans lequel une amplification des au moins deux première et seconde molécules d'ARN adénylées différentes a lieu pendant ou après l'adénylation selon l'étape d).

8. Procédé selon une des revendications précédentes, dans lequel une dégradation des première et seconde molécules d'ARN est effectuée par une ribonucléase après réalisation de l'hybridation selon les étapes f) et g) ainsi que du prolongement de brin selon les étapes h) et i).

9. Procédé selon une des revendications précédentes, dans lequel le premier et le second nucléoside triphosphate sont marqués avec le même colorant de fluorescence.

10. Procédé selon une des revendications précédentes, dans lequel le colorant de fluorescence est un colorant de cyanine, notamment Cy3.

11. Kit de réalisation d'un procédé selon une des revendications 1 à 10 pour le dépistage différencié d'au moins deux première et seconde molécules d'ARN différentes, présentes dans une solution d'échantillon, qui présentent chacune une séquence polynucléotidique identique A, contenant :
a1) un microréseau nucléotidique comprenant au moins deux premier et second contre-ZIP différents, immobilisés à différentes positions sur le support de microréseau nucléotidique,
b1) un polynucléotide amorce-cZIP X présentant une séquence nucléotidique d'amorce cA et une séquence nucléotidique cZIP X, dans lequel la séquence nucléotidique d'amorce cA est complémentaire à une section en 5' terminale de la séquence polynucléotidique A et dans lequel la séquence nucléotidique cZIP X est complémentaire au premier contre-ZIP du microréseau nucléotidique, et
c1) un polynucléotide amorce-cZIP Y présentant la séquence nucléotidique d'amorce cA et une séquence nucléotidique cZIP Y et dans lequel la séquence nucléotidique cZIP Y est complémentaire au second contre-ZIP du microréseau nucléotidique, et
contenant une solution I qui contient au moins un premier nucléoside triphosphate marqué avec un colorant de fluorescence et une solution II qui contient au moins un second nucléoside triphosphate marqué avec un colorant de fluorescence, dans lequel le premier nucléoside triphosphate est sélectionné parmi le groupe constitué du désoxy-uridine triphosphate et désoxy-thymidine triphosphate, et dans lequel le second nucléoside triphosphate est sélectionné parmi le groupe constitué du désoxy-guanosine triphosphate, désoxy-adénosine triphosphate et désoxy-cytidine triphosphate.

12. Kit selon la revendication 11, dans lequel le kit contient au moins une enzyme pour une polyadénylation et au moins une enzyme pour le prolongement de brin.
